(19) Europäisches Patentamt
European Patent Office
Office européen des brevets

(11) EP 1 347 063 A1

(12) # EUROPEAN PATENT APPLICATION

(43) Date of publication:
**24.09.2003 Bulletin 2003/39**

(21) Application number: **02006257.6**

(22) Date of filing: **20.03.2002**

(51) Int Cl.[7]: **C12Q 1/68**, A61K 31/00, A61K 38/00, C12N 15/63, C12N 5/10, C07K 16/18, A01K 67/027

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Biofrontera Pharmaceuticals AG**
**51377 Leverkusen (DE)**

(72) Inventors:
- **Lübbert, Hermann, Prof.Dr.**
  **51381 Leverkusen (DE)**
- **Engels, Peter, Dr.**
  **51429 Bergisch Gladbach (DE)**
- **Zwilling, Stefan, Dr.**
  **51375 Leverkusen (DE)**

(74) Representative:
**Sternagel, Fleischer, Godemeyer & Partner**
**Patentanwälte**
**Braunsberger Feld 29**
**51429 Bergisch Gladbach (DE)**

(54) **Multiple genes relevant for the characterisation, diagnosis and manipulation of stroke**

(57) The present invention relates to the use of differentially expressed polynucleotide sequences or polypeptides for the characterisation of stroke or progression thereof or progression of neurodegenerative processes as consequence thereof, a method for characterising stroke, a method for identifying therapeutic agents for stroke and the use of such sequences for the development of a medicament.

EP 1 347 063 A1

## Description

The present invention relates to the use of differentially expressed polynucleotide sequences or polypeptides for the characterisation of stroke or progression thereof or progression of neurodegenerative processes as consequence thereof, a method for characterising stroke, a method for identifying therapeutic agents for stroke and the use of such sequences for the development of a medicament.

Stroke is a rather common and potentially harmful event that often leaves patients severely disabled for the rest of their lives. It is the result of either an interruption of blood and therefore oxygen supply to the brain, or bleeding in the brain that occurs when a blood vessel bursts. The reduced oxygen supply leads to shortage of energy in the affected brain regions and results in the death of neurons around the lesion. The degeneration spreads from the site that is affected by the reduced blood supply into regions which have at all times obtained sufficient oxygen.

Stroke results from a loss of blood flow to the brain caused by thrombosis or haemorrhage. With an incidence of 250-400 in 100.000 and a mortality rate of around 30%, stroke is a major public health problem. About one-half of the stroke survivors suffer from significant persisting neurological impairment and/or physical disability. Thus, the economic costs of stroke amount to many billions of dollars worldwide.

A stroke occurs when the blood supply to part of the brain is suddenly interrupted or when a blood vessel in the brain bursts. As a consequence, brain cells die when they no longer receive oxygen and nutrients from the blood or when they are damaged by sudden bleeding into the brain. Some brain cells die immediately after interruption of the blood flow into the brain, while others remain at risk for death and stay in a compromised state for hours. These damaged cells make up the so-called "ischemic penumbra", and with timely treatment these cells could be saved. Stroke ultimately leads to infarction, the death of huge numbers of brain cells, which are eventually replaced by a fluid-filled cavity (or infarct) in the injured brain.

There are two major forms of stroke: ischemic - blockage of a blood vessel supplying the brain, and hemorrhagic - bleeding into or around the brain. An ischemic stroke can be caused by a blood clot (embolus or thrombus), which is blocking a vessel. It can also be caused by the narrowing of an artery due to the build-up of plaque (a mixture of fatty substances, including cholesterol and other lipids). The underlying pathological process called stenosis is often observed in arteriosclerosis, the most common blood vessel disease. About 80% of all strokes are ischemic strokes. A hemorrhagic stroke is caused by the bursting of an artery in the brain. Subsequently, blood spews out into the surrounding tissue and upsets not only the blood supply but also the delicate chemical balance neurons require to function. Hemorrhagic strokes account for approximately 20% of all strokes.

A transient ischemic attack (TIA), sometimes called a mini-stroke, starts just like a stroke but then resolves leaving no noticeable symptoms or deficits. The occurrence of a TIA is a warning that the person is at risk for a more serious and debilitating stroke. About one-third of patients who have a TIA will have an acute stroke sometime in the future. The addition of other risk factors compounds a person's risk for a recurrent stroke. The average duration of a TIA is a few minutes. For almost all TIAs, the symptoms go away within an hour. There is no possibility to distinguish whether symptoms will be just a TIA or persist and lead to death or disability.

Recurrent stroke is frequent; about 25 percent of people who recover from their first stroke will have another stroke within 5 years. Recurrent stroke is a major contributor to stroke disability and death, with the risk of severe disability or death from stroke increasing with each stroke recurrence. The risk of a recurrent stroke is greatest right after a stroke, with the risk decreasing with time. About 3 percent of stroke patients will have another stroke within 30 days of their first stroke and one-third of recurrent strokes take place within 2 years of the first stroke.

The most important risk factors for stroke are hypertension, arteriosclerosis, heart disease, diabetes, and cigarette smoking. Others include heavy alcohol consumption, high blood cholesterol levels, illicit drug use, and genetic or congenital conditions, particularly vascular abnormalities. People with multiple risk factors compound the destructive effects of these risk factors and create an overall risk greater than the simple cumulative effect of the individual risk factors.

Although stroke is a disease of the brain, it can affect the entire body. Depending on the affected brain region and the severity of the attack, post-stroke patients suffer from a variety of different symptoms. Some of the disabilities that can result from stroke include paralysis, cognitive deficits, speech problems, emotional difficulties, daily living problems, and pain. Stroke disability is devastating to the stroke patient and family, but therapies are available to help rehabilitate post-stroke patients. The mortality rate observed in ischemic stroke is around 30%. The time window for a medical treatment is narrow and limited to anticoagulants and thrombolytic agents, which must be given immediately (at latest 3 hours) after having a stroke. Unfortunately, there is no effective neuroprotective medication available, which is able to stop the delayed degeneration of neurons following the initial stroke attack.

Stroke symptoms appear suddenly. The following acute symptoms can be observed: Sudden numbness of the face, arm or leg, difficulties in talking or understanding speech, trouble seeing in one or both eyes, sudden trouble in walking, loss of balance and coordination. Severe headache with no known cause does also occur. Even more importantly, there is a variety of severe disabilities occurring and persisting in post-stroke patients. Paralysis is a frequent disability resulting from stroke. Cognitive deficits (problems with thinking, awareness, attention and learning) are also commonly observed. Post-stroke patients exhibit language deficits and also emotional deficits (like post-stroke depression). Furthermore, an uncommon type of pain, called central pain syndrome (CPS), can occur after having a stroke.

Currently the only effective treatment for thrombotic stroke is the use of anticoagulants (e.g. heparin), and thromobolytics (recombinant tissue plasminogen activator). Neuroprotective agents, which are effective in animal models, have generally proved ineffective in the clinic, and none are yet registered for use in stroke.

A number of experimental models have been developed for global ischemia and focal ischemia. The availability of these different models provides an opportunity to investigate mechanisms of stroke. Finding common features in different models or over several time points within one model should provide better insight into the mechanisms critical for stroke, and comparison of the models should help to understand development, progression and consequences of stroke.

Most common global ischemia models are

a) the two vessel model

Models of transient global ischemia resulting in patterns of selective neuronal vulnerability are models that attempt to mimic the pathophysiology of cardiac arrest or hemodynamic conditions that result from severe systemic hypotension. Reversible high-grade forebrain ischemia is generated by bilateral common carotid artery (CCA) occlusion. Together with systemic hypotension conditions it reduces the blood flow to severe ischemic levels (Smith et al. 1984, Acta Neuropathol. 64; p319-332). This model of transient global ischemia has the advantage of a one stage surgical preparation, the production of a high-grade forebrain ischemia and the possibility to conduct chronic survival studies in order to assess the potential of neuroprotective drugs.

b) the four vessel model

This model results in a high-grade forebrain ischemia but is produced in two stages, one to manipulate each of the CCA, and the second stage 24h later to produce the forebrain ischemia. The advantage is that the second step can be produced in awake freely moving animals (Pulsinelli et al. 1982, Ann.Neurol. 11; 491-498). Similar pathohistological results could be obtained for both vessel models.

c) the cardiac arrest model

Forebrain ischemia models are of value to study cerebral ischemia but these models do not exactly mimic the hemodynamic consequences of a cardiac arrest, which results in a complete ischemia of the brain, spinal cord and extracerebral organs (Katz et al. 1995; J. Cereb. Blood Flow Metab. 15; 1032-1039). The initial cardiac arrest models from Safar et al. (1982, Protection of tissue against hypoxia; Elsevier Biomedical Press; 147-170 ) or Korpaczew et al. (1982, Partol Fizjol Eksp Ter 3; 78-80), were developed further by Katz et al. (1989, Resuscitation 17; 39-53) and Pluta et al. (1991, Acta Neuropathologica 83; 1-11) to models with controllable insult. Katz and colleagues (1995) have reported a reproducible outcome model of cardiac arrest with apneic asphyxia of 8 min, leading to the cessation of circulation at 3-4 min of apnea and resulting in cardiac arrest of 4-5 min. At 72 hr after injury, widespread patterns of ischemic neurons were found in many brain regions, including cerebral cortex, caudate putamen CA1 and CA3 regions of hippocampus, thalamus, cerebellum and brain stem.

Pluta et al. described a primary mechanical cardiac arrest model whereby global ischemia was induced by cardiac arrest for 3 to 10 min with survival periods of the animals from 3 min to 7 days.

Although these models have several limitations, they provide a method for studying the mechanisms of neuronal injury resulting from the clinically realistic cerebral insult and screening potential cerebral resuscitation therapies.

Focal Ischemia models

Models of permanent or transient focal ischemia typically giving rise to localised brain infarction have routinely been used to investigate the pathophysiology of stroke. For example, models of middle cerebral artery (MCA) occlusion in a variety of species have gained increased acceptance due to their relevance to the human clinical setting.

a) the permanent MCA Occlusion models

Tamura and colleagues (1981, J. Cereb. Blood Flow Metab. 1; 53-60) developed a subtemporal approach as standard model of proximal MCA occlusion. In models of permanent MCA occlusion, electrocauterisation of the MCA proximal to the origin of the lateral lenticulostriate arteries is utilised routinely. In these models, severe reductions in blood flow are seen within the ischemic core, with milder reductions in blood flow within the border or penumbral regions. The addition of moderate arterial hypotension has the effect of enlarging infarct volume.

b) the transient MCA Occlusion models

In human ischemic stroke, recirculation frequently occurs after focal ischemia. Thus, models of transient MCA occlusions have also been developed, mainly in rats or mice, whereby surgical clip or sutures are introduced to induce a transient ischemic insult.

Some further animal models for stroke are considered in several reviews like the articles of W.D. Dietrich (1998; Int. Review of Neurobiology, Vol.42; 55-101) , Wiebers et al. (1990 Stroke 21; 1-3) or Zivin and Grotta (1990 Stroke 21; 981-983).

Object of the present invention is to identify and characterise development, conditions (status which elicits), progression and consequences of stroke on a molecular basis.

This object is met by the use of polynucleotide sequences selected from the group of sequences SEQ ID NO: 1 to 88 or homologues or fragments thereof or the according polypeptides for the characterisation of a) development and /or occurrence of stroke, b) the progression of the pathology of stroke and / or b) the consequences of the pathology of stroke, whereby the characterisation is carried out outside of a living body.

Polynucleotide sequences SEQ ID NO.1 to 88 are expressed sequence tags (ESTs) representing genes, which are differentially expressed under stroke, particularly under global ischemia in the cardiac arrest model (Pluta et al, 1991 Acta Neuropathol. 83; 1-11).

The model is explained in more detail in the literature and in the examples below.

The term "polynucleotide sequence" or "nucleic acid sequence" designates in the present application any DNA or RNA sequence, independent of the length. Thus this term can describe short sequences like PCR primers or probes for hybridisation, as well as whole genes or cDNA of these genes.

The term "polypeptide" or "amino acid sequence" designates a chain of amino acids, independent of their length, however, in any case more than one amino acid.

As "homologues" of polynucleotide sequences such polynucleotide sequences are designated which encode the same type of protein as one of the polynucleotide sequences described herein. Accordingly as "homologues" of a polypeptide the polypeptides are designated, which have an amino acid sequence, wherein at least 70 %, preferably 80 %, more preferably 90 % of the amino acids are identical to one of the proteins of the present invention and wherein the replaced amino acids preferably are replaced by homologous amino acids. As "homologous" amino acids are designated those, which have similar features concerning hydrophobicity, charge, steric features etc.. Most preferred are amino acid sequences, containing the species- or family-dependent differences of the amino acid sequence. Particularly as "homologues" sequences are designated those, which correspond to one of the cited sequences in another species or individual. For example if in the present invention a rat model is used and the cited polynucleotide sequence encodes the rat protein, the according polynucleotide sequence and protein of a mouse or of a human is designated as "homologue". Further splice variants and members of gene families are designated as homologues.

"Fragments" of a polynucleotide sequence are all polynucleotide sequences, which have at least 10 identical base pairs compared to one of the polynucleotide sequences shown in the present application or by the genes represented by these polynucleotide sequences. The term "fragment" encloses therefore such fragments as primers for PCR, probes for hybridisation, DNA fragments included in DNA vectors like plasmids, cosmids, BACs or viral constructs, as well as shortened splice variants of the genes identified herein. As a fragment of a protein (polypeptide) amino acid sequences are designated which have at least three amino acids, preferably at least 10 amino acids. Therefore fragments serving as antigens or epitopes are enclosed in this designation.

In the present application the term "sequence" is used when either a polynucleotide sequence (= nucleic acid sequence) or a polypeptide (= amino acid sequence) or a protein is meant. That means, when it is irrelevant which type of sequence is used the type is not designated particularly, but with the more common term "sequence".

In the present application the term "stroke" means the development, occurrence, progression and consequences of the disease state. Several features of the development, occurence and consequences of this disease are described herein above.

The basis of the models and methods described in the present application is the examination and determination of the expression of genes, which are differentially expressed during development, conditions, progression and consequences of stroke. Therefore for the examination each sequence can be used which allows the determination of the expression rate of the considered gene. Such a sequence can be at least one of the polynucleotide sequences SEQ ID NO. 1 to 88 or homologues or fragments thereof, as well as the polypeptides encoded thereby, however, just as well polynucleotide sequences and the according polypeptides can be used which are (parts of) the genes represented by the polynucleotide sequences SEQ ID NO. 1 to 88.

According to the invention it has been found, that the genes represented by the polynucleotide sequences SEQ ID NO. 1 to 88 are differentially expressed in the cardiac arrest model of stroke.

Therefore the present invention provides sequences, which represent genes, which are differentially expressed under stroke. Such polynucleotide sequences and the according polypeptides allow the determination and examination of stroke. Most of these sequences have not yet been regarded in relation to stroke. Sequences which are known to be differentially expressed in connection with stroke conditions are Apolipoprotein E, herein referred to as SEQ ID NO: 40, (J. Cereb. Blood Flow Metab 2001 Oct; 21(10): 1199-1207), β-amyloid precursor protein (APP), herein referred to as SEQ ID NO: 77 (Neuroreport 1996 Nov 4; 7(15-17): 2727-2731), Preproenkephalin, herein referred to as SEQ ID NO: 35 (Brain Res. 1997 Jan 2; 744(1): 185-187), Cathepsin B, herein referred to as SEQ ID NO: 86 (J. Neurosurg. 1997 Nov; 87(5): 716-723).

For these examinations animal models can be used. As such a model any animal can be used wherein the necessary preparations can be carried out, however mammalian models are preferred, even more preferred are rodents. Most preferred animal models of the present invention are rat and mouse models.

The sequences of the present invention further can be used for diagnosing stroke of a human outside of the living body by determining the expression levels of at least one of the cited sequences in comparison to the non-disease status. During treatment period of a patient the expression of the presently shown sequences can also be used outside of the body for assessing the efficacy of stroke treatment. In this case blood, cerebrospinal fluid (CSF) or tissue is removed from the patient and expression is determined in the samples.

For determination and comparison of the expression levels of at least one of the genes identified in the present invention any of the commonly known methods can be used, either on RNA/cDNA level or on protein level. For example PCR, hybridisation, micro array based methods, western blot or 2-D protein gel analysis are suitable methods. One preferred method is the digital expression pattern display method (DEPD method), explained in detail in WO99/42610. The method used for determination of expression levels is not restrictive, as long as expressed amounts can be quantified.

The sequences of the present invention can further be used to develop new animal models for stroke. By examination of the expression levels of at least one of the shown sequences, a procedure might be determined, which is useful for generating a suitable animal model for different interesting conditions. In particular, useful animal models might be transgenic, knock out, or knock in models.

In such a newly generated animal model as well as in one of the known models the efficacy of compounds can be tested, using techniques known in the art. As well assay systems can be used that are based on the shown sequences. Such assay systems may be *in vivo, ex vivo* or *in vitro* assays. In any case the models or assay systems are contacted with the compound(s) to be tested and samples are obtained from these models / systems, wherein expression levels of the sequences are determined and compared to the non-treated model / system.

Dependent of the model used the samples can be derived from whole blood, CSF or whole tissue, from cell populations isolated from tissue or blood or from single cell populations (i.e. cell lines).

In one embodiment of the invention cellular assays can be used. Preferred cells for cellular assays are eukaryotic cells, more preferably mammalian cells. Most preferred are neuronal-like cells, like SHSY5Y (neuroblastoma cell line), hippocampal murine HT-22 cells, primary cultures from astrocytes, cerebral cortical neuronal-astrocytic co-cultures, mixed neuronal/glial hippocampal cultures, cerebellar granular neuronal cell cultures, primary neuronal cultures derived from rat cortex (E15-17), or COS cells (African green monkey, kidney cells); CHO cells (Chinese hamster ovary), or HEK-293 cells (human embryonic kidney).

Whereas the comparison of the expression levels (disease / non-disease status) of at least one of the provided sequences might give information about the examined disease status, it is preferred to determine the expression levels of more than one of the sequences simultaneously. Thus several combinations of the sequences can be used at different time points. By combination of several sequences a specific expression pattern can be determined indicating and / or identifying the conditions of the disease. The more expression rates are determined simultaneously, the more specific the result of the examination might be. However, good results also can be obtained by combination of only a few sequences. Therefore for the present invention it is preferred to compare the expression rates of at least two of the sequences provided herein, more preferred of at least 4, further more preferred of at least 6 of the sequences.

Since the presently provided sequences represent genes, which are differentially expressed, the expression rates of the single genes can be increased or decreased independently from each other. "Independently" in this context means that the expression rate of each of the genes can but need not be influenced by each other. In any case expression levels different from the non-disease status might be a hint to the disease status, which is examined.

The disease status, which is considered in the present invention, is stroke. The preferred types of stroke are ischemic and hemorrhagic stroke. Consequences, which might be related to stroke, are among others severe headache, paralysis, cognitive deficits, speech problems, emotional difficulties, daily living problems, and central pain syndrome.

Independent whether stroke is diagnosed or characterised, a model for stroke is characterised, the efficacy of stroke treatment or the efficiency of a compound in a model shall be examined, the determination of the expression levels of at least one of the sequences is carried out outside of a living body. A method to obtain such results comprises

a) providing a sample comprising cells or body fluids expressing one or more genes represented by polynucleotide sequences selected from the group of SEQ ID NO. 1 to 88 or homologues or fragments thereof
b) detecting expression of one or more of the genes in said cells;
c) comparing the expression of the genes in the test cells to the expression of the same genes in reference cells whose expression stage is known, and
d) identifying a difference in expression levels of the considered sequences, if present, in the test cell population and the reference cell population.

As mentioned above, detection of the expression of the genes can be carried out by any method known in the art. The method of detection is not limiting the invention.
Expression levels can be detected either on basis of the polynucleotide sequences or by detecting the according polypeptide, encoded by said polynucleotide sequence.
Preferred methods for detection and determination of the gene expression levels are PCR of cDNA, generated by reverse transcription of expressed mRNA, hybridisation of polynucleotides (Northern, Southern Blot systems, *In situ* hybridisation), DNA-microarray based technologies, detection of the according peptides or proteins via e.g. Western Blot systems, 2-dimensional gel analysis, protein micro-array based technologies or quantitative assays like e.g. ELISA tests.
The most preferred method for quantitative analysis of the expression levels is the digital expression pattern display method (DEPD), described in detail in WO99/42610.
The sequences of the present invention can further be used for identifying therapeutic agents and their efficacy for treating stroke. For example a method can be used comprising:

a) providing a test cell population comprising cells capable of expressing one or more genes represented by nucleic acid sequences selected from the group consisting of SEQ ID NO: 1 to 88 or homologues or fragments thereof
b) contacting said test cell population with the test therapeutic agent;
c) detecting the expression of one or more of the genes in said test cell population,
d) comparing the expression of the gene(s) in the test cell population to the expression of the gene(s) in a reference cell population whose disease stage is known; and
e) identifying a difference in expression levels of the considered sequences, if present, in the test cell population and the reference cell population, thereby identifying a therapeutic agent for treating stroke.

Test cells can be obtained from a subject, an animal model or cell cultures of fresh cells or cell lines. Further *in vitro* assays may be used.
A method examining the different expression patterns of the differentially expressed gene(s) therefore can be used for testing agents and compounds for their efficiency for treatment of stroke. Which model is used is not relevant, as long as the model allows the determination of differences in expression amounts.
In such a model cells are contacted with the interesting agent or compound and expression of at least one of the genes considered in the present invention is determined in comparison to the expression of the same gene in cells which never have been contacted to the according agent / compound. Contacting the cells either can be affected by administering the agent /compound to an animal or by contacting isolated cells of tissue, CSF, or blood or cells of cell lines in culture with the agent / compound.
By examination of the influence the considered agent(s) / compound(s) have on the expression of at least one of the genes, the efficacy of the agent(s) / compound(s) can be estimated. This allows the decision whether it is worthwhile to develop a medicament containing such an agent or compound.
Whether the expression is determined on basis of mRNA generation or on basis of protein generation is not relevant, as long as the difference of the expression rate can be determined. Therefore both, the polynucleotide sequences, and the polypeptides or proteins shown in the present application can be used for the development or the identification of a medicament.
The development of a medicament can be desirable for example if the considered compound / agent has any influence on the regulation of the expression rate or on the activity of any polynucleotide sequence or polypeptide /protein of the present invention. Said influence can be for example acceleration, promotion, increase, decrease or inhibition of the expression or activity.
Said influence of a compound or agent can be examined by a method comprising contacting a sample comprising one of the nucleic acid sequences or of the polypeptides of the present invention with a compound that binds to said sequence in an amount sufficient to determine whether said compound modulates the activity of the polynucleotide or polypeptide / protein sequence.

By such a method a compound or agent modulating the activity of any of the nucleic acid sequences or any polypeptides of the present invention can be determined.

Furthermore the sequences itself can be used as a medicament.

An example for such a use is the use of a polynucleotide sequence as an antisense agent. Antisense agents, including but not limited to ribonucleotide or desoxyribonucleotide oligomers, or base-modified oligomers like phosphothioates, methylated nucleotides, or PNAs (peptide nucleic acids), can hybridise to DNA or mRNA, inhibiting or decreasing transcription or translation, respectively. Thus, polynucleotide sequences of a gene, which is increased in expression rate under stroke, can be used as antisense agents to decrease the expression rates of said gene. Further such polynucleotide sequences can be used for gene therapy.

Another example for such a use is the use of a polypeptide or a protein as a medicament. In case that the expression of a gene is decreased under stroke and therefore not "enough" protein is provided by the body to maintain natural (healthy) conditions, said protein can be administered as a medicament. In case a gene is increased under stroke, representing a protective beneficial or adaptive response of the brain, this effect can be further strengthened by adding even more of the corresponding protein as medicament.

A pharmaceutical composition comprising a polynucleotide sequence or a polypeptide according to the present invention can be any composition, which can serve as a pharmaceutical one. Salts or aids for stabilising the sequences in the composition preferably are present.

For the determination of the expression of the relevant genes the generated sequences have to be detected. Therefore several reagents can be used, which are for example specific radioactive or non-radioactive (e.g. biotinylated or fluorescent) probes to detect nucleic acid sequences by hybridisation e.g. on DNA microarrays, primer sets for the detection of one or several of the nucleic acid sequences by PCR, antibodies against one of the polypeptides, or epitopes, or antibody- or protein-microarrays. Such reagents can be combined in a kit, which can be sold for carrying out any of the described methods. The isolated polynucleotide or polypeptide sequences therefore can be provided as microarrays ("DNA chips" and "protein chips").

Further the sequences defined in the present invention can be used to "design" new transgenic animals as model for stroke. Therefore the animals are "created" by manipulating the genes considered in the present application in a way that their expression in the transgenic animal differs from the expression of the same gene in the wild type animal. In which direction the gene expression has to be manipulated (up- or down-regulation) depends on the gene expression shown in the present application. Methods of gene manipulation and methods for the preparation of transgenic animals are commonly known to those skilled in the art.

For further examinations or experiments it might be desirable to include any of the nucleic acids of the present invention into a vector or a host cell. By including the sequences in a host cell for example cellular assays can be developed, wherein the genes, polynucleotide sequences and the according proteins / polypeptides further can be used or examined. Such vectors, host cells and cellular assays therefore shall be considered as to fall under the scope of the present invention.

Figures:

Figures 1 to 7 show results of several transcripts differentially expressed in the cardiac arrest model over several time points. Each sequence is examined in their expression levels over a time period of 0.5 hrs after cardiac arrest to 2 years after surgery and compared to sham operated controls. Genes are described as differentially expressed when the sequence is up- or down-regulated at one or more time points with a certain statistical relevant significance value. Over time the expression pattern can be determined as up-regulated in one to seven time points; as down regulated in one to seven time points or as mixed regulated if the type of regulation changes between up- and down-regulation at different time points.

X-axis describes the time points analysed by DEPD, 1= 0.5hrs post operation, 2= 3hrs post operation, 3= 6hrs post operation, 4=day 3 post operation, 5=day 7 post operation, 6= 2 years post operation.

The Y-axis shows delta h, which represents the normalised difference of expression (peak height) of a certain transcript between a control group and a treated group. x-fold difference in gene expression is calculated by:

$$1 + \text{delta h} /1 - \text{delta h}$$

0= no change to control, + = up regulation, - = down regulation; (0,2 = 1,5 fold; 0,3 = 1,86 fold; 0,4 = 2,33 fold; 0,5 = 3 fold).

The following examples are provided for illustration and are not intended to limit the invention to the specific example provided.

Examples:

Example 1: Preparation of rat cardiac arrest model

Cardiac arrest was performed in female Lewis-rats at 3 months of age (150-220g), resulting in total cessation of blood flow leading to global cerebral ischemia. After 10 min of ischemia, the animals were resuscitated by external heart massage and ventilation. The group size was 2 x 3 animals. For induction of cardiac arrest, a special blunt-end, hook-like probing device was inserted into the right parasternal line across the third "intracostal spaces into the chest cavity. Next, the probe was gently pushed down the vertrebral column until a slight resistance from the presence of the right pulmonary veins was detected. The probe then was tilted 10-20° caudally. Then, the probe was rotated in a counter-clockwise direction about 135-140° under the inferior vena cava. At this position, the occluding part of the device was positioned right under the heart vessle bundle (inferior vena cava, superior right and left vena cava, ascending aorta, and pulmonary trunk). The pulmonary veins (left and right) were closed by the rotation of the occluding part of the hook. In the last step, the probe was pulled up with concomitant compression of the heart vessel bundle against the sternum. The end of the occluding portion of the hook was then positioned in the left parasternal line in the second intercostal space. To prevent upward movement of the chest and to insure complete ligation of the vessels, simple finger pressure was applied downward the sternum, producing total hemostasis and subsequent ventricular arrest. The effect of the whole procedure is total cessation of both arterial and venous blood flow, it essentially represents the onset of clinical death. After 2,5-3,5 min, the probe was released and removed from the chest by reverse procedural succession, and the animals remained in this position until the beginning of resuscitation.
The resuscitation procedure consisted of external heart massage until spontaneous heart function was recovered and controlled respiration occured. During this time, air was pumped through a polyethylene tube inserted intratracheally that was connected to a respirator. External heart massage was produced by the the index and middle fingers rapidly striking against the chest (sternum) for 1-2 min at the level of the fourth intracostal area with a frequency of 150-240/min in continuous succession. The ratio of strikes to frequency of ventilation was 6:1 or 8:1.
An electrocardiogramm (lead II-EEG) was recorded continuously during the course of the experiment. Moreover, heart activity was monitored using a loud speaker connected to the output lead of the electroencephalograph. Additionally, the cranial bones were exposed at the sagittal and coronal sutures, where silver-needle electrodes were attached for recording on an electrocorticogramm (ECoG). All measurements were registered on a ten-channel electroencephalogramm (Accutrace-100A, Beckmann).
2 x 3 sham operated animals served as controls. These animals were treated similarly to the experimental group with one major exception. Under anesthesia, the probe was inserted through the chest wall into the plural cavity as has already been described above but without further manipulation and torsion of the probe. The probe remained in the chest for essentially the same time period (3,5 min) as in the experimental group. The control animals were then returned to their cages for recovery.
Tissue preparation occurred 0.5hr, 1hr, 6hrs, 3 days, 7 days and 2 years after surgery. Tissues were frozen on liquid nitrogen prior to RNA preparation.

Example 2: Determination of expression levels

Gene expression profiling by DEPD-analysis starts with the isolation of 5-10µg total RNA. In a second step, double-stranded cDNA is synthesized. Through an enzymatic digest of the cDNA with three different type IIS restriction enzymes, three pools with short DNA-fragments containing single-stranded overhangs are generated. Afterwards, specific DNA-adaptor-molecules are ligated and in two subsequent steps 3.072 PCR reactions are performed by using 1024 different unlabelled 5'primer and a common FAM-fluorescent- labelled 3'-primer in the last PCR step. Subsequently, the 3072 PCR pools are analysed on an automatic capillary electrophoresis sequencer.
Differential gene expression pattern of single fragments are determined by comparison of normalized chromatogram peaks from the control groups and corresponding operated animals.

Example 3: Sequencing and Databank analysis of the obtained sequences

Differentially expressed peaks are confirmed on polyacrylamid gels by using radioactive labelled 3' primer instead of the FAM fluorescent primer. Differentially expressed bands are cut from the gel. After an elution step of up to 2 hrs in 60µl 10mM Tris pH8, fragments are re-amplified by PCR using the same primer as used in the DEPD analysis. Resulting PCR products are treated with a mixture of ExonucleaseI and shrimp alkaline phosphatase prior to direct sequencing. Sequencing reactions are performed by using DY-Enamic-ET-dye terminator sequencing kit (Amersham) and subsequently analysed by capillary electrophoresis (Megabace 1000, Amersham).
Prior to a BLAST sequence analysis (Altschul et al. 1997, Nucleic Acids Res. 25:3389-3402) against Genbank (Release No. 126), all sequences are quality verified and redundant sequences or repetitive motifs are masked.

Results are shown in Table 1.

| Seq-ID | ascession number | name | fragment length [bp] |
|---|---|---|---|
| 1 | Z99755 | Human DNA sequence from clone CTA-714B7 on chromosome 22q12.2-13.2 Contains pseudo-gene similar to part of COX7B (cytochrome c oxidase subunit VIIb) | 323 |
| 2 | | no hit found in DB | 323 |
| 3 | BF418899 | UI-R-BJ2-bqk-c-03-0-UI.s1 UI-R-BJ2 Rattus norvegicus cDNA clone UI-R-BJ2-bqk-c-03-0-UI 3', mRNA sequence | 323 |
| 4 | | no hit found in DB | 138 |
| 5 | AB023781 | Rattus norvegicus mRNA for cathepsin Y, partial cds | 301 |
| 6 | BF420410 | UI-R-BJ2-bqb-g-04-0-UI.s1 UI-R-BJ2 Rattus norvegicus cDNA clone UI-R-BJ2-bqb-g-04-0-UI 3', mRNA sequence | 258 |
| 7 | M35826 | Rat mitochondrial NADH-dehydrogenase (NDI) gene, complete cds | 375 |
| 8 | AK019199 | Mus musculus 11 days embryo cDNA, RIKEN full-length enriched library, clone:2700005I17, full insert sequence | 221 |
| 9 | BF413204 | -UI-R-BT1-bny-a-04-0-UI.s1 UI-R-BT1 Rattus norvegicus cDNA clone UI-R-BT1-bny-a-04-0-UI 3', mRNA sequence | 251 |
| 10 | X16555 | Rat PRPS2 mRNA for phosphoribosylpyrophosphate synthetase subunit II | 208 |
| 11 | AK017685 | Mus musculus 8 days embryo cDNA, RIKEN full-length enriched library, clone:5730466L18, full insert sequence | 238 |
| 12 | | no hit found in DB | 201 |
| 13 | Y00964 | M musculus mRNA for beta-hexosaminidase | 217 |
| 14 | | no hit found in DB | 145 |
| 15 | L17127 | Rattus norvegicus proteasome RN3 subunit mRNA, complete cds | 311 |
| 16 | | no hit found in DB | 171 |
| 17 | BG380139 | UI-R-CS0-btp-e-04-0-UI.s1 UI-R-CS0 Rattus norvegicus cDNA clone UI-R-CSO-btp-e-04-0-UI 3', mRNA sequence | 171 |
| 18 | D86215 | Rattus norvegicus mRNA for NADH:ubiquinone oxidoreductase, complete cds | 325 |
| 19 | AK005320 | Mus musculus adult male cere-bellum cDNA, RIKEN full-length enriched library, clone:1500031J01, full insert sequence | 171 |
| 20 | | no hit found in DB | 171 |
| 21 | | no hit found in DB | 199 |

(continued)

| Seq-ID | ascession number | name | fragment length [bp] |
|---|---|---|---|
| 22 | | no hit found in DB | 171 |
| 23 | | no hit found in DB | 139 |
| 24 | | no hit found in DB | 139 |
| 25 | BI287855 | UI-R-CWOs-ccm-a-07-0-UI.s1 UI-R-CW0s Rattus norvegicus cDNA clone UI-R-CWOs-ccm-a-07-0-UI 3', mRNA sequence | 269 |
| 26 | | no hit found in DB | 166 |
| 27 | | no hit found in DB | 227 |
| 28 | AC016673 | Homo sapiens BAC clone RP11-17N4 from 2, complete sequence | 261 |
| 29 | AB071989 | Mus musculus mRNA for Spop. partial cds | 365 |
| 30 | | no hit found in DB | 248 |
| 31 | AF178845 | Rattus norvegicus calmodulin mRNA, complete cds | 482 |
| 32 | J02701 | Rat Na+, K+ -ATPase beta sub-unit protein mRNA, complete cds | 418 |
| 33 | X12553 | Rat mRNA for liver cytochrome c oxidase subunit VIa | 444 |
| 34 | | no hit found in DB | 140 |
| 35 | Y07503 | Rat mRNA for preproenkephalin (A) | 244 |
| 36 | X14876 | Rat mRNA for transthyretin | 245 |
| 37 | | no hit found in DB | 270 |
| 38 | AI547530 | UI-R-C3-sr-b-11-0-UI.s1 UI-R-C3 Rattus norvegicus cDNA clone UI-R-C3-sr-b-11-0-UI 3', mRNA sequence | 207 |
| 39 | M27315 | Rattus norvegicus cytochrome c oxidase subunit II (Co II) gene | 188 |
| 40 | J02582 | Rat apolipoprotein E gene, complete cds | 180 |
| 41 | | no hit found in DB | 250 |
| 42 | U65579 | Human mitochondrial NADH de-hydrogenase-ubiquinone Fe-S protein 8, 23 kDa subunit precursor (NDUFS8) nuclear mRNA encoding mitochondrial protein, complete cds | 337 |
| 43 | AF173082 | Mus musculus LIN-7 homolog 2 (MALS-2) mRNA, complete cds | 158 |
| 44 | | no hit found in DB | 250 |
| 45 | U70268 | Rattus norvegicus mud-7 mRNA, 3' UTR | 241 |
| 46 | X96997 | O.aries SOX-2 gene | 187 |
| 47 | AK020957 | Mus musculus adult male corpora quadrigemina cDNA, RIKEN full-length enriched library, clone:B230104P22, full insert sequence | 221 |
| 48 | | no hit found in DB | 490 |
| 49 | BC012314 | Mus musculus, Similar to ferritin heavy chain, clone MGC:19422 IMAGE:3488821, mRNA, complete cds | 116 |

(continued)

| Seq-ID | ascession number | name | fragment length [bp] |
|---|---|---|---|
| 50 | BB452941 | BB452941 RIKEN full-length enriched, 12 days embryo spinal ganglion Mus musculus cDNA clone D130020J05 3', mRNA sequence | 159 |
| 51 | AK019418 | Mus musculus 13 days embryo head cDNA, RIKEN full-length enriched library, clone:3110018K01, full insert sequence | 377 |
| 52 | J01435 | Rattus norvegicus mitochondrial ATPase subunit 6 gene | 224 |
| 53 | BF387893 | UI-R-CA1-bbw-a-03-0-UI.s1 UI-R-CA1 Rattus norvegicus cDNA clone UI-R-CA1-bbw-a-03-0-UI 3', mRNA sequence | 200 |
| 54 | AB033713 | Rattus norvegicus mitochondrial gene for cytochrome b, partial cds | 149 |
| 55 | | no hit found in DB | 154 |
| 56 | U53513 | Rattus norvegicus glycine-, glutamate-, thienylcyclohexylpiperidine-binding protein mRNA, complete cds | 162 |
| 57 | AK004546 | Mus musculus adult male lung cDNA, RIKEN full-length enriched library, clone:1200002H13, full insert sequence | 301 |
| 58 | | no hit found in DB | 304 |
| 59 | AY004290 | Rattus norvegicus scg10-like-protein mRNA, complete cds | 321 |
| 60 | | no hit found in DB | 306 |
| 61 | | no hit found in DB | 155 |
| 62 | BF544005 | UI-R-E0-ce-c-04-0-UI.r1 UI-R-E0 Rattus norvegicus cDNA clone UI-R-E0-ce-c-04-0-UI 5', mRNA sequence | 155 |
| 63 | | no hit found in DB | 276 |
| 64 | | no hit found in DB | 306 |
| 65 | | no hit found in DB | 186 |
| 66 | BC011132 | Mus musculus. Similar to special AT-rich sequence binding protein 1, clone MGC:18461 IMAGE:4164993, mRNA, complete cds | 239 |
| 67 | AJ278701 | Rattus norvegicus mRNA for cytosolic branched chain aminotransferase (Bcatc gene) | 233 |
| 68 | M14512 | Rat Na+,K+-ATPase alpha(+) isoform catalytic subunit mRNA, complete cds | 246 |
| 69 | U42975 | Rattus norvegicus Shal-related potassium channel Kv4.3 mRNA, complete cds | 309 |
| 70 | BC004706 BC004706 | Mus musculus, heterogeneous nuclear ribonucleoprotein C, clone MGC:5715 IMAGE: 3499283, mRNA, complete cds | 336 |

(continued)

| Seq-ID | ascession number | name | fragment length [bp] |
|---|---|---|---|
| 71 | BE101398 | UI-R-BJ1-aud-e-10-0-UI.s1 UI-R-BJ1 Rattus norvegicus cDNA clone UI-R-BJ1-aud-e-10-0-UI 3', mRNA sequence | 190 |
| 72 | AF073297 | Mus musculus small EDRK-rich factor 2 (Serf2) mRNA, complete cds | 180 |
| 73 | D32249 | Rattus norvegicus mRNA for neurodegeneration associated protein 1, complete cds | 302 |
| 74 | BF391228 | UI-R-CA1-bcq-g-07-0-UI.sl UI-R-CA1 Rattus norvegicus cDNA clone UI-R-CA1-bcq-g-07-0-UI 3', mRNA sequence | 166 |
| 75 | BE109851 | UI-R-CAO-axi-b-04-0-UI.sl UI-R-CA0 Rattus norvegicus cDNA clone UI-R-CA0-axi-b-04-0-UI 3', mRNA sequence | 205 |
| 76 | | no hit found in DB | 163 |
| 77 | AY011335 | Rattus norvegicus amyloid beta precursor protein (App) gene, partial cds | 282 |
| 78 | BC013540 | Mus musculus, Similar to retinal short-chain dehydrogenase/reductase 1, clone MGC:19224 IMAGE:4241608, mRNA, complete cds | 246 |
| 79 | X52311 | Rat unr mRNA for unr protein with unknown function | 335 |
| 80 | M29358 | Rat ribosomal protein S6 mRNA, complete cds | 232 |
| 81 | AC068987 | UI-R-CAO-bkh-f-06-0-UI.sl UI-R-CA0 Rattus norvegicus cDNA clone UI-R-CA0-bkh-f-06-0-UI 3', mRNA sequence | 369 |
| 82 | AC068987 | UI-R-B01-aqb-b-02-0-UI.s1 UI-R-B01 Rattus norvegicus cDNA clone UI-R-B01-aqb-b-02-0-UI 3', mRNA sequence | 381 |
| 83 | | no hit found in DB | 219 |
| 84 | M23953 | UI-R-BJ0p-aio-h-09-0-UI.s1 UI-R-BJ0p Rattus norvegicus cDNA clone UI-R-BJ0p-aio-h-09-0-UI 3', mRNA sequence | 360 |
| 85 | AK018721 | Mus musculus adult male kidney cDNA, RIKEN full-length enriched library, clone:0610007M20, full insert sequence | 488 |
| 86 | X82396 | R. norvegicus mRNA for cathepsin B | 457 |
| 87 | | no hit found in DB | 215 |
| 88 | X82550 | R.norvegicus mRNA for ribosomal protein L41 | 249 |
| DB = data base | | | |

Example 4: Comparison of differentially expressed sequences over several time points in the cardiac arrest model.

0.5hr, 1hr, 3hrs, 6hrs, 3days, 7 days, and 2 years survival time of the animals were chosen as time points for gene expression profiling of the cardiac arrest model. After DEPD analysis peaks obtained as differentially expressed at least at one time point were compared over time to control within the cardiac arrest stroke model. Results are shown in Table 2.

Table 2

| Seq-ID No. | Regulation |
|---|---|
| 1 | down 7d |
| 2 | down 7d |
| 3 | down 7d |
| 4 | down 7d |
| 5 | up 3d / up 7d |
| 6 | up 3d |
| 7 | up 3d |
| 8 | down 3d |
| 9 | down 3d |
| 10 | up 3d |
| 11 | down 3d |
| 12 | up 3d |
| 13 | up 3d |
| 14 | up 7d |
| 15 | up 7d |
| 16 | up 3d |
| 17 | down 7d |
| 18 | up 7d |
| 19 | down 7d |
| 20 | down 7d |
| 21 | up 7d |
| 22 | up 3d / down 7d |
| 23 | up 3d |
| 24 | down 3d |
| 25 | down 7d |
| 26 | up 3d |
| 27 | down 3d |
| 28 | up 3d |
| 29 | down 3d |
| 30 | down 3d |
| 31 | down 7d |
| 32 | up 3d |
| 33 | up 7d |
| 34 | up 3d |
| 35 | up 3d / up 7d |
| 36 | up 7d |
| 37 | up 7d |

Table 2 (continued)

| Seq-ID No. | Regulation |
|---|---|
| 38 | down 7d |
| 39 | up 7d |
| 40 | up 7d |
| 41 | down 7d |
| 42 | up 7d |
| 43 | up 7d |
| 44 | down 7d |
| 45 | down 7d |
| 46 | up 7d |
| 47 | up 7d |
| 48 | down 7d |
| 49 | up 3d |
| 50 | down 7d |
| 51 | down 7d |
| 52 | up 7d |
| 53 | up 3d |
| 54 | up 3d |
| 55 | up 3d |
| 56 | up 3d |
| 57 | down 3d |
| 58 | down 3d |
| 59 | down 3d |
| 60 | down 3d |
| 61 | up 3d |
| 62 | down 3d |
| 63 | up 3d |
| 64 | up 3d |
| 65 | up 3d |
| 66 | up 3d / down 7d |
| 67 | up 3d |
| 68 | up 3d |
| 69 | down 3d |
| 70 | down 3d / down 7d |
| 71 | up 3d |
| 72 | up 3d |
| 73 | up 3d |
| 74 | down 3d |
| 75 | down 2y |

Table 2 (continued)

| Seq-ID No. | Regulation |
|---|---|
| **76** | up 2y |
| **77** | up 2y |
| **78** | down 2y |
| **79** | down 2y |
| **80** | down 2y |
| **81** | up 2y |
| **82** | up 2y |
| **83** | up 2y |
| **84** | up 2y |
| **85** | up 2y |
| **86** | down 2y |
| **87** | up 2y |
| **88** | up 2y |

For each DNA fragment, gene expression patterns are obtained in the stroke model compared over several time points. "Up", "down", and "mixed" is defined as time dependent expression at one or more time points in the cardiac arrest model compared to the non-disease model.

## Claims

1. Use of polynucleotide sequences selected from the group of sequences SEQ ID NO: 1 to 34, 36 to 39, 41 to 76, 78 to 85 and 87, 88 or homologues or fragments thereof or the according polypeptides for the characterisation of the development and/or occurrence of stroke and / or the progression of the pathology of stroke and/or consequences of stroke, whereby the characterisation is carried out outside of a living body.

2. Use of a combination of at least two of polynucleotide sequences selected from the group of sequences SEQ ID NO: 1 to 88 or homologues or fragments thereof or the according polypeptides for the characterisation of the development and/or occurrence of stroke and / or the progression of the pathology of stroke and/or consequences of stroke, whereby the characterisation is carried out outside of a living body.

3. Use of polynucleotide sequences according to claim 1 or 2, **characterised in that** the sequences are mammalian sequences.

4. Method for characterising a stroke status, comprising the comparison of the expression of genes represented by at least one of the sequences selected of the group of SEQ ID NO. 1 to 34, 36 to 39, 41 to 76, 78 to 85 and 87, 88 or homologues or fragments thereof to non-disease status.

5. Method for characterising a stroke status, comprising the comparison of the expression of genes represented by at least two of the sequences selected of the group of SEQ ID NO.1 to 88 or homologues or fragments thereof to non-disease status.

6. Method according to claim 4 or 5, for diagnosing stroke outside of the living body.

7. Method according to claim 4 or 5, for assessing the efficacy of stroke treatment outside of a living body.

8. Method according to claim 4 or 5, for characterisation of animal models for stroke.

9. Method according to claim 4 or 5, for determining the efficiency of compounds in a model for stroke.

10. Method according to any of claims 4 to 9, whereby the expression is determined from in vivo samples, in vitro samples, or ex vivo samples.

11. Method according to claim 10, whereby the samples are derived from whole tissues, cerebrospinal fluid (CSF) or blood, or from cell populations isolated from tissues, CSF, or blood, or from cell lines.

12. Method according to any of claims 4 to 11, whereby at least a combination of 4 sequences are compared to non-disease status.

13. Method according to any of claims 4 to 11, whereby at least a combination of 6 sequences are compared to non-disease status.

14. Method according to any of claims 4 to 13, whereby the considered gene expression is increased compared to non-disease status.

15. Method according to any of claims 4 to 13, whereby the considered gene expression is decreased compared to non-disease status.

16. Method according to any of claims 4 to 13, whereby the considered gene expression for at least one sequence is increased and for at least one sequence is decreased compared to non-disease status.

**17.** The method according to any of claims 4 to 16 comprising:

a) providing a sample comprising cells expressing one or more genes represented by polynucleotide sequences selected from the group of SEQ ID NO. 1 to 88 or homologues or fragments thereof
b) detecting expression of one or more of the genes in said cells;
c) comparing the expression of the genes in the test cells to the expression of the same genes in reference cells whose expression stage is known, and
d) identifying a difference in expression levels of the considered sequences, if present, in the test cell population and the reference cell population.

**18.** A method of identifying a test therapeutic agent for treating stroke in a subject, the method comprising:

a) providing a test cell population comprising cells capable of expressing one or more genes represented by nucleic acid sequences selected from the group consisting of SEQ ID NO: 1 to 88 or homologues or fragments thereof
b) contacting said test cell population with the test therapeutic agent;
c) detecting the expression of one or more of the genes in said test cell population,
d) comparing the expression of the gene(s) in the test cell population to the expression of the gene(s) in a reference cell population whose disease stage is known; and
e) identifying a difference in expression levels of the considered sequences, if present, in the test cell population and the reference cell population, thereby identifying a therapeutic agent for treating stroke.

**19.** Method according to claims 4 to 18, whereby the gene expression is considered or determined by PCR of cDNA, hybridisation of a sample DNA or by detecting the according protein.

**20.** Use of at least one of the polynucleotide sequences of genes represented by polynucleotide sequences selected from SEQ ID NO. 1 to 88 or homologues or fragments thereof or of a protein encoded by any gene represented by any of the polynucleotide sequences selected from SEQ ID NO. 1 to 88 or homologues or fragments thereof for the development or identification of a medicament.

**21.** Use according to claim 20, whereby the medicament changes the expression or the activity of a protein encoded by a gene represented by one of said polynucleotide sequences.

**22.** Use according to claim 20 or 21 for the development of a medicament for the treatment of stroke.

**23.** Use or method according to any of claims 1 to 18 or 22, whereby the stroke is ischemic or hemorrhagic stroke.

**24.** Combination of isolated nucleic acid sequences comprising at least one nucleic acid sequence of a gene that is one of the genes represented by the polynucleotide sequences selected of SEQ ID NO. 1 to 88, or the complement of the nucleic acid sequence as an microarray.

**25.** Combination of isolated polypeptides comprising at least one amino acid sequence encoded by one of the genes represented by the nucleic acid sequences of SEQ ID NO. 1 to 88.

**26.** Use of an isolated nucleic acid sequence of a gene that is one of the genes represented by the polynucleotide sequences selected of SEQ ID NO. 1 to 88 or the according isolated polypeptide as a medicament.

**27.** A pharmaceutical composition comprising any of the nucleic acid sequences of a gene that is one of the genes represented by the polynucleotide sequences selected of SEQ ID NO. 1 to 88 or any of the according polypeptides.

**28.** A kit comprising one or more reagents for detecting one or more genes represented by nucleic acid sequences selected from the group consisting of SEQ ID NO. 1 to 88 or homologues or fragments thereof.

**29.** A vector comprising any nucleic acid sequence of of a gene that is one of the genes represented by the polynucleotide sequences selected of SEQ ID NO. 1 to 88.

**30.** A host cell comprising any isolated nucleic acid sequence of a gene that is one of the genes represented by the polynucleotide sequences selected of SEQ ID NO. 1 to 88 or a vector of claim 29.

**31.** An antibody that selectively binds to a polypeptide encoded by a of a gene that is one of the genes represented by the polynucleotide sequences selected of SEQ ID NO. 1 to 88, and fragments, homologues, analogues and derivatives of the antibody.

**32.** A transgenic animal wherein at least one of the sequences corresponding to the sequences 1 to 88 is manipulated in the animal in comparison to the wild type.

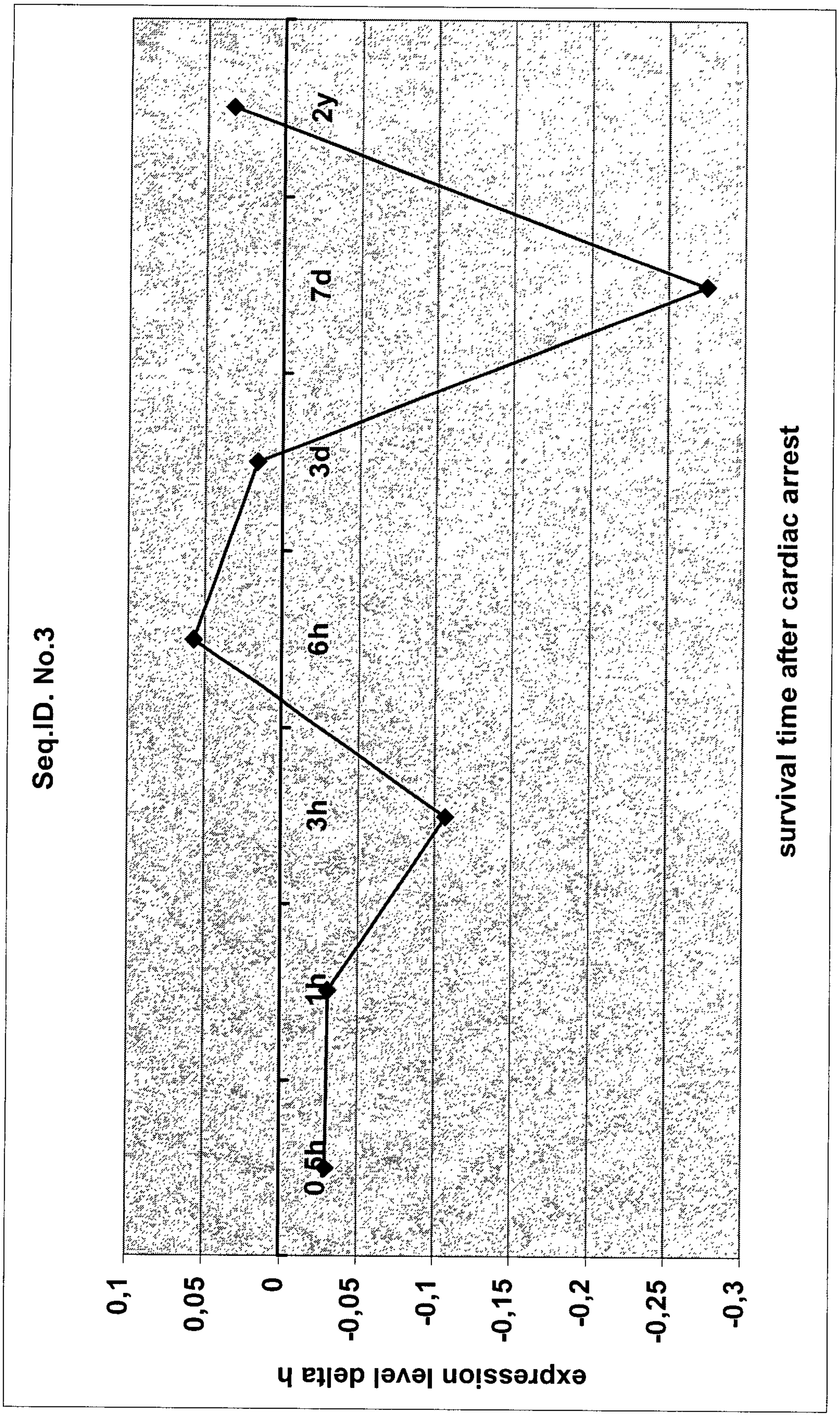
Seq.ID. No.3
expression level delta h
0,1
0,05
0
-0,05
-0,1
-0,15
-0,2
-0,25
-0,3
0.5h
1h
3h
6h
3d
7d
2y
survival time after cardiac arrest

Figure 1

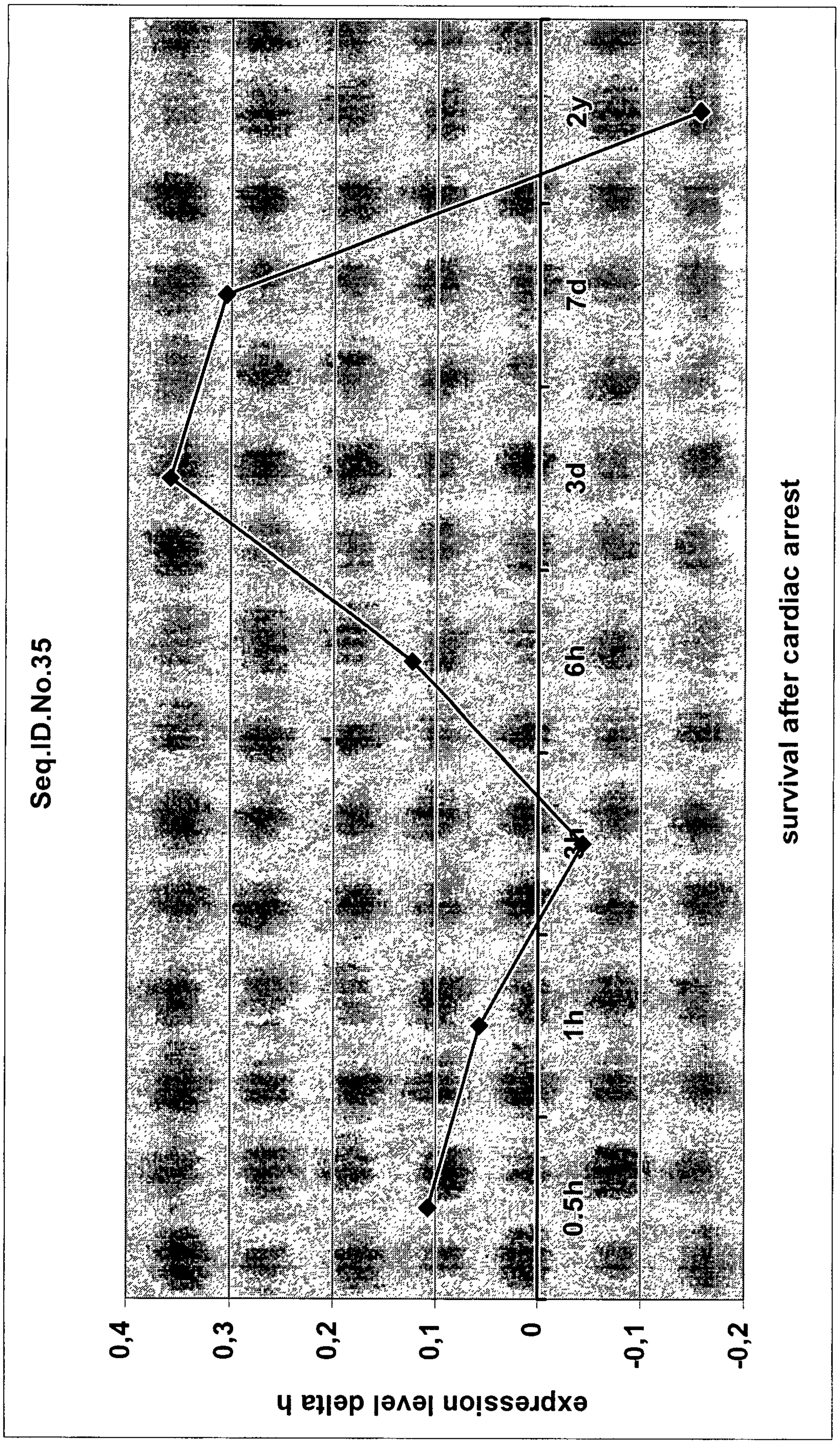
Seq.ID.No.35
expression level delta h
0,4
0,3
0,2
0,1
0
-0,1
-0,2
0.5h
1h
3h
6h
3d
7d
2y
survival after cardiac arrest

Figure 2

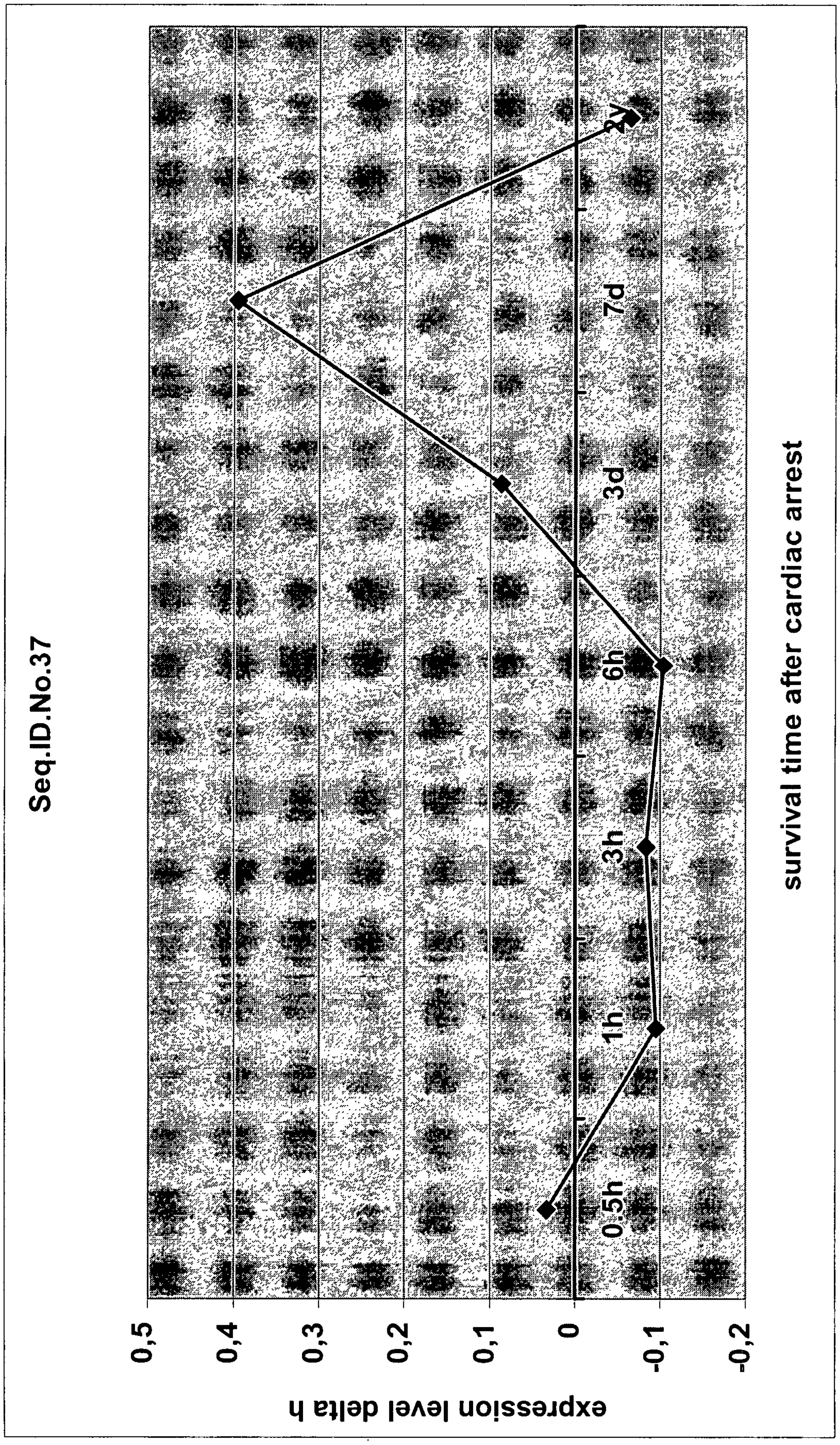
Seq.ID.No.37
expression level delta h
0,5
0,4
0,3
0,2
0,1
0
-0,1
-0,2
0.5h
1h
3h
6h
3d
7d
2y
survival time after cardiac arrest

Figure 3

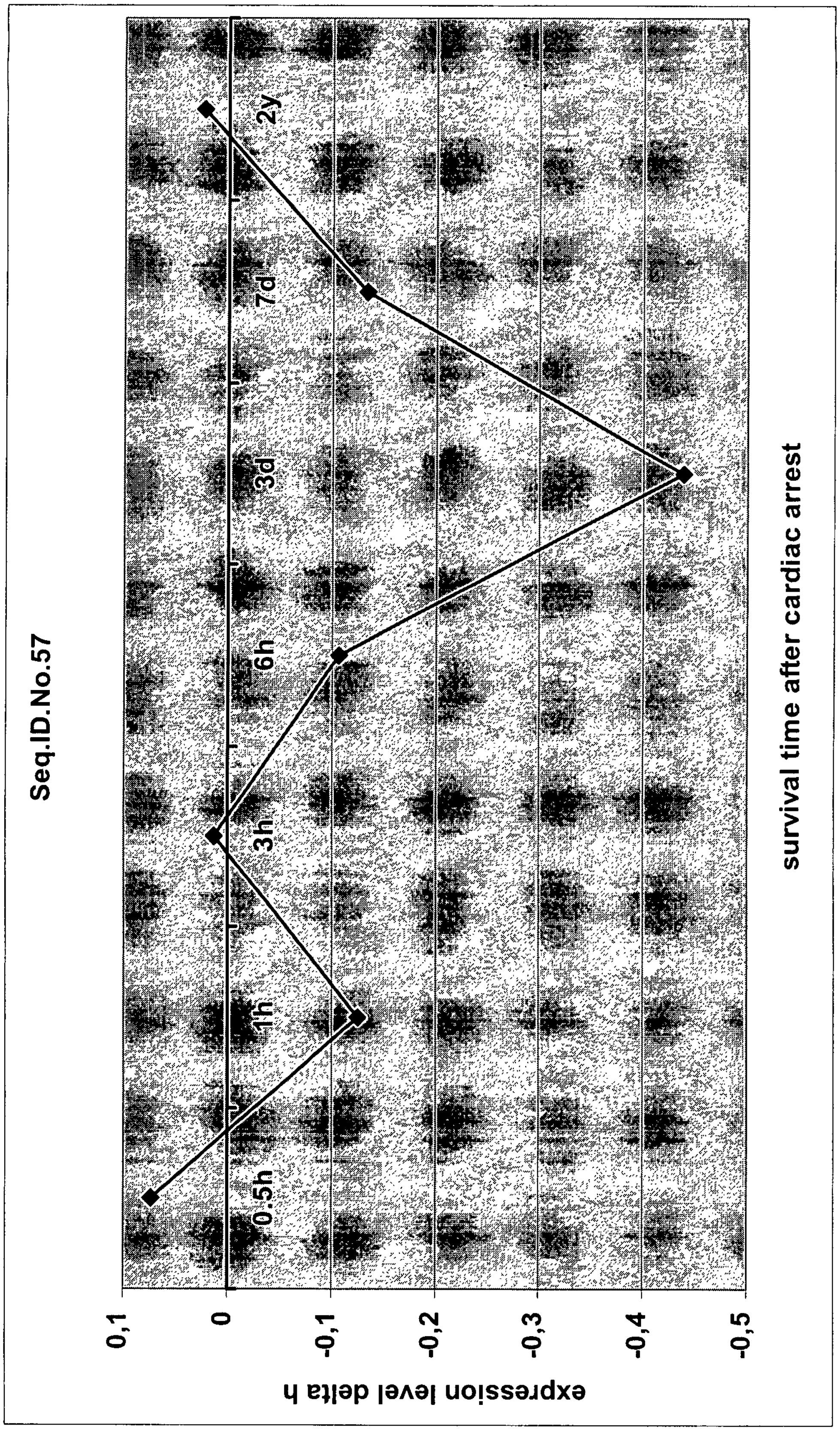

Seq.ID.No.57
expression level delta h
0,1
0
-0,1
-0,2
-0,3
-0,4
-0,5
0.5h
1h
3h
6h
3d
7d
2y
survival time after cardiac arrest


Figure 4

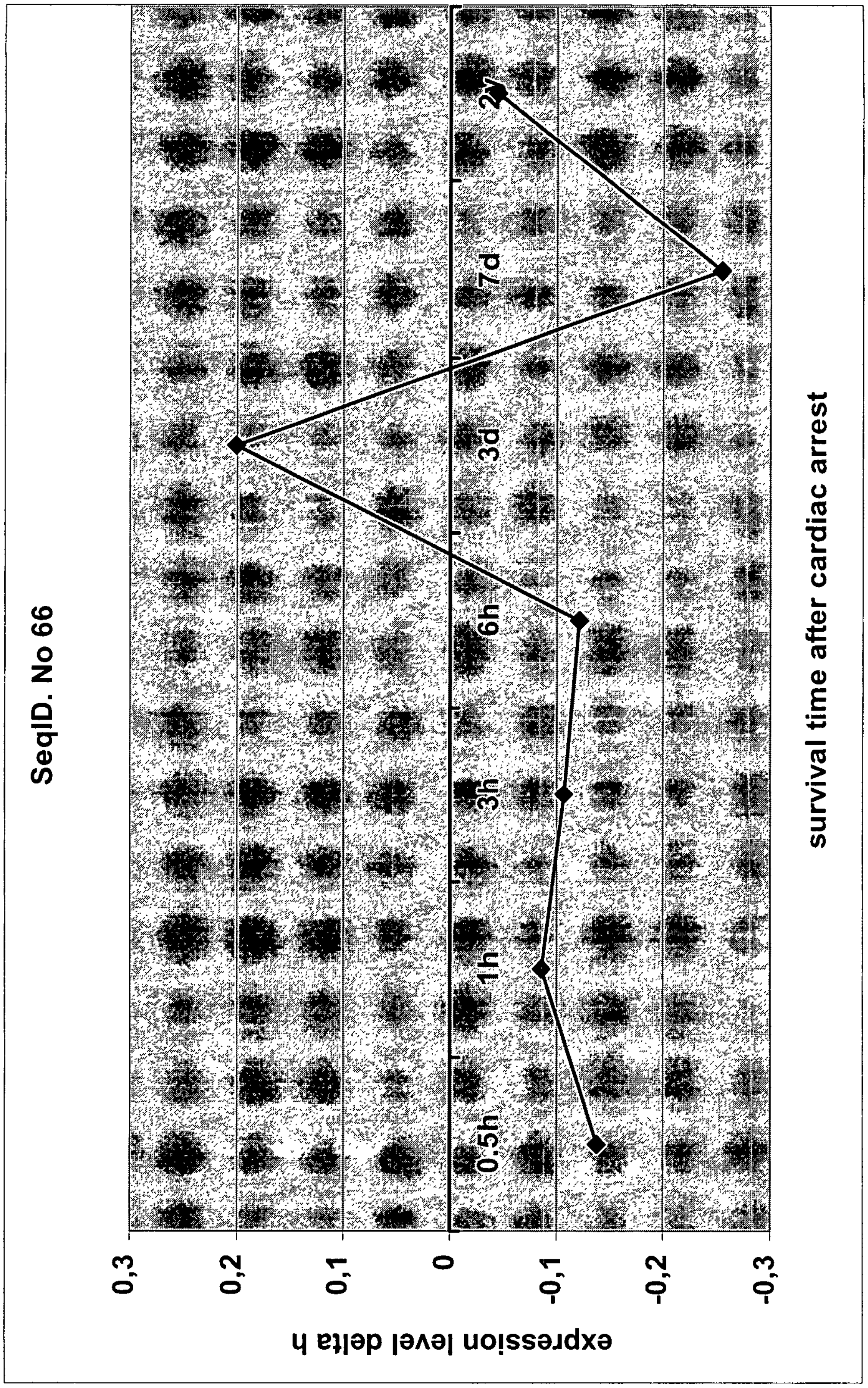
SeqID. No 66
expression level delta h
0,3
0,2
0,1
0
-0,1
-0,2
-0,3
0.5h
1h
3h
6h
3d
7d
survival time after cardiac arrest

Figure 5

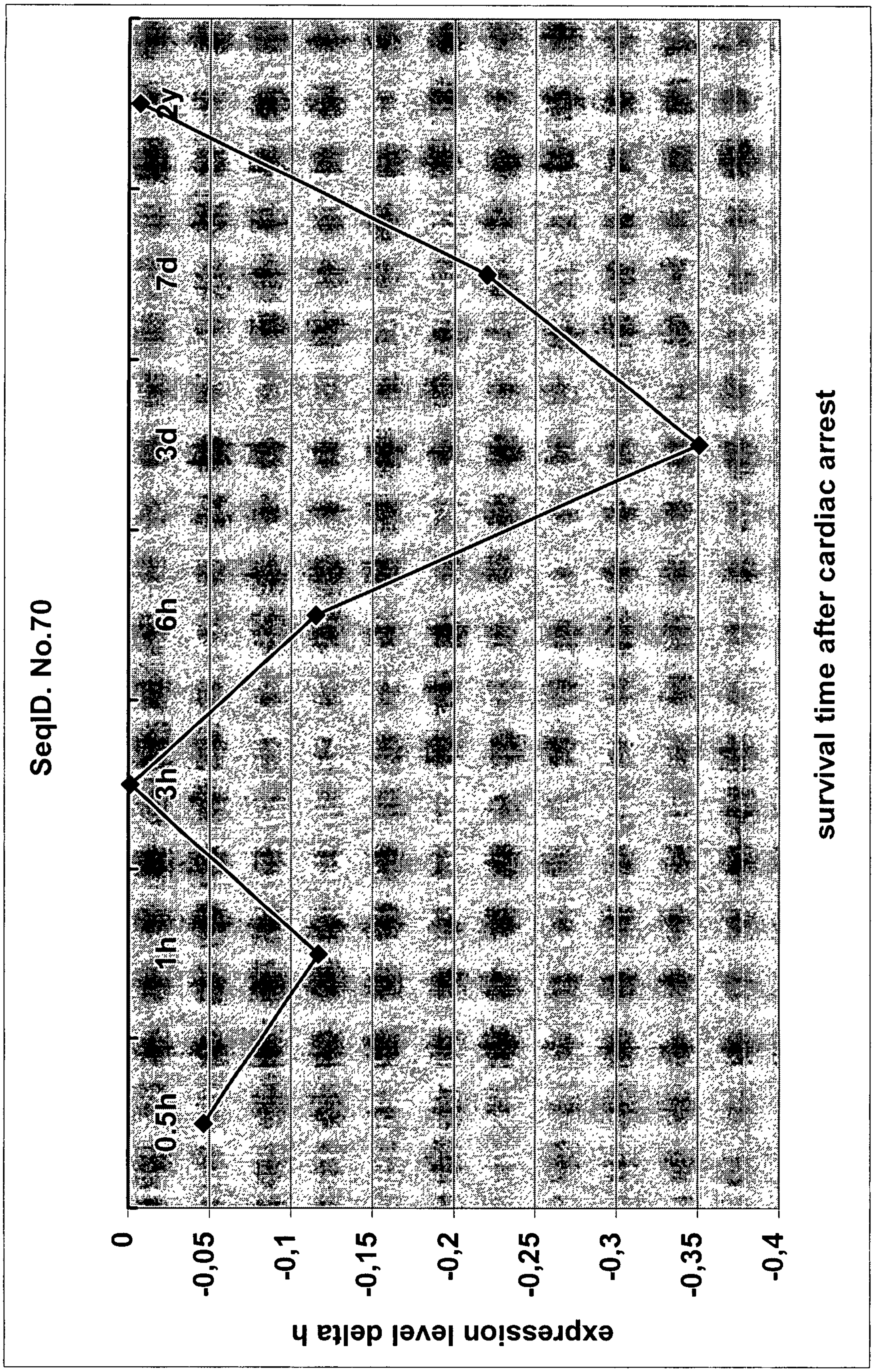
SeqID. No.70
0.5h
1h
3h
6h
3d
7d
2y
0
-0,05
-0,1
-0,15
-0,2
-0,25
-0,3
-0,35
-0,4
expression level delta h
survival time after cardiac arrest

Figure 6

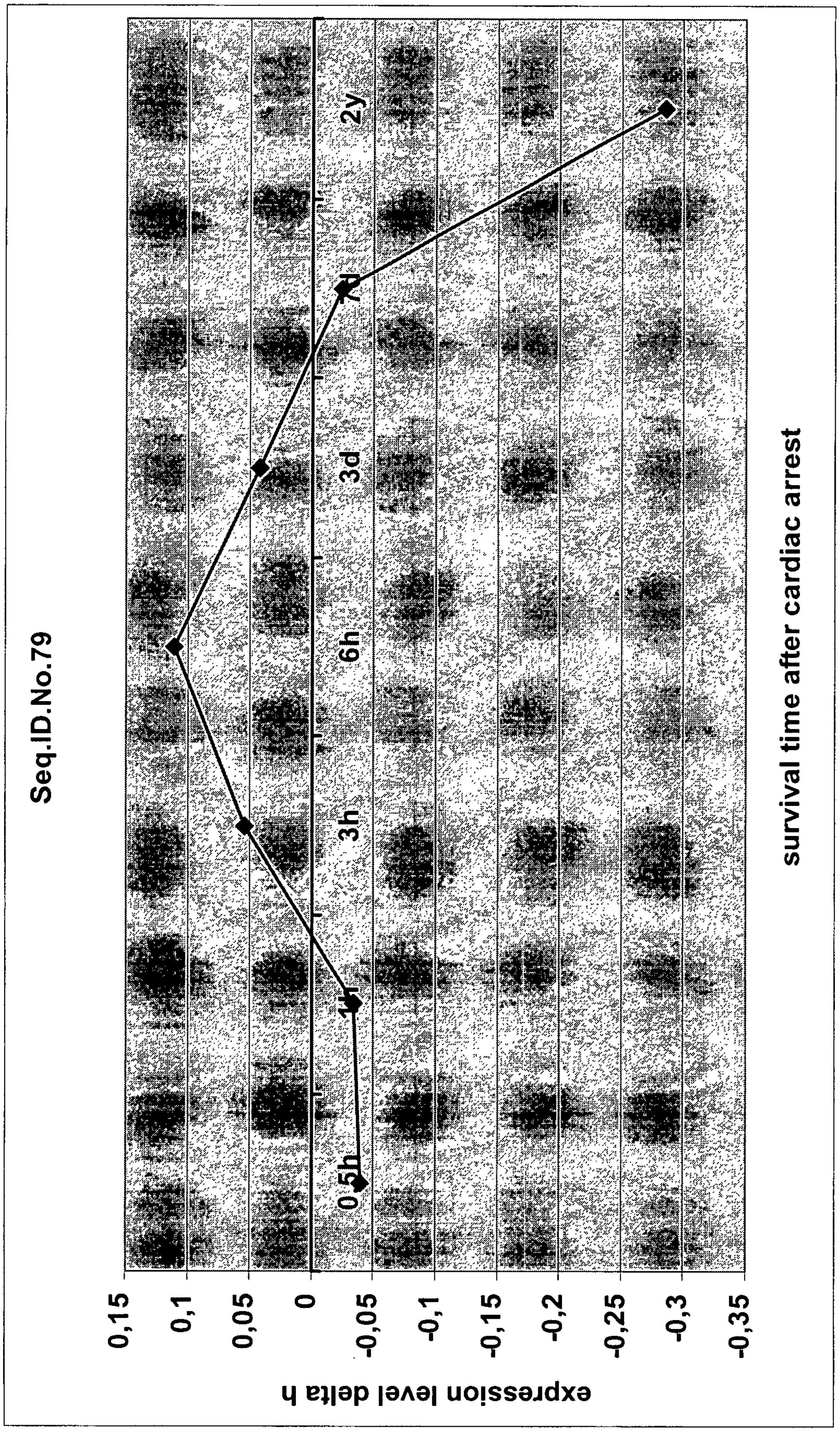
Seq.ID.No.79
0,15
0,1
0,05
0
-0,05
-0,1
-0,15
-0,2
-0,25
-0,3
-0,35
expression level delta h
0,5h
3h
6h
3d
2y
survival time after cardiac arrest

Figure 7

European Patent Office

**PARTIAL EUROPEAN SEARCH REPORT**

which under Rule 45 of the European Patent Convention *shall be considered, for the purposes of subsequent* proceedings, as the European search report

Application Number
EP 02 00 6257

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | SHAMLOO M ET AL: "Comprehensive gene expression analysis in focal cerebral ischemia." SOCIETY FOR NEUROSCIENCE ABSTRACTS, vol. 27, no. 1, 2001, page 538 XP001109085 31st Annual Meeting of the Society for Neuroscience; San Diego, California, USA; November 10-15, 2001 ISSN: 0190-5295 * the whole document * --- | 1-32 | C12Q1/68<br>A61K31/00<br>A61K38/00<br>C12N15/63<br>C12N5/10<br>C07K16/18<br>A01K67/027 |
| Y | MICHAEL D B ET AL: "Gene expression following rat forebrain ischemia by microarray assay and immunocytochemistry." SOCIETY FOR NEUROSCIENCE ABSTRACTS, vol. 27, no. 1, 2001, page 572 XP001109084 31st Annual Meeting of the Society for Neuroscience; San Diego, California, USA; November 10-15, 2001 ISSN: 0190-5295 * the whole document * --- | 1-32 | |
| | -/-- | | **TECHNICAL FIELDS SEARCHED (Int.Cl.7)**<br>C12Q |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

*Reason for the limitation of the search:*

see sheet C

| Place of search | Date of completion of the search | Examiner |
|---|---|---|
| THE HAGUE | 7 March 2003 | Pinta, V |

CATEGORY OF CITED DOCUMENTS

X : particularly relevant if taken alone
Y : particularly relevant if combined with another *document of the same category*
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 02 00 6257

Claim(s) searched incompletely:
32

Reason for the limitation of the search (non-patentable invention(s)):

Article 53 (a) EPC - Contrary to "ordre public" or morality
Under Art. 53 (a) EPC taken in combination with Rule 23d EPC, the subject-matter of claim 32 related to a transgenic animal not expressly excluding human is not patentable. Accordingly, the search has been limited to non-human transgenic animals.

--------------------

Further limitation of the search

Claim(s) searched completely:
1-3

Claim(s) searched incompletely:
4-31

Reason for the limitation of the search:

Present claims 4-31 relate to (a nucleic acid sequence of) a gene or polypeptide(s) encoded by the same that is "represented by" SEQ ID NO:1, or homologues or fragments thereof. However, the present application fails to comply with the requirements for support within the meaning of Article 84 EPC and/or disclosure within the meaning of Article 83 EPC since it does not provide the sequence of such a gene, nor is such a gene known from the prior art (cf. sequence homology search, Table 1). In these conditions no meaningful search can be conducted as to portions of said gene or polypeptide(s) encoded by the same other than those comprising SEQ ID NO:1 or the corresponding polypeptide.

Consequently, the terms "represented by" have been construed as "comprising" for the purpose of search. Therefore the search has been carried out for those parts of the application which do appear to be clear (and concise), namely on the basis of nucleic acid sequences comprising SEQ ID NO:1 or polypeptide(s) encoded by the same.

European Patent Office

# PARTIAL EUROPEAN SEARCH REPORT

Application Number
EP 02 00 6257

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| Y | SCHNEIDER A ET AL: "A systematic approach towards identifying novel drug targets in models of cerebral ischemia."<br>SOCIETY FOR NEUROSCIENCE ABSTRACTS,<br>vol. 27, no. 1, 2001, page 540 XP001109087<br>31st Annual Meeting of the Society for Neuroscience; San Diego, California, USA; November 10-15, 2001<br>ISSN: 0190-5295<br>* the whole document *<br>--- | 1-32 | |
| Y | READ S J ET AL: "STROKE GENOMICS: APPROACHES TO IDENTIFY, VALIDATE, AND UNDERSTAND ISCHEMIC STROKE GENE EXPRESSION"<br>JOURNAL OF CEREBRAL BLOOD FLOW AND METABOLISM, RAVEN PRESS, LTD., NEW YORK, NY, US,<br>vol. 21, no. 7, July 2001 (2001-07), pages 755-778, XP001057074<br>ISSN: 0271-678X<br>* the whole document *<br>--- | 1-32 | TECHNICAL FIELDS SEARCHED (Int.Cl.7) |
| Y | WO 99 42610 A (NOVARTIS ERFIND VERWALT GMB; NOVARTIS AG (CH); HOFFMANN RALF (DE))<br>26 August 1999 (1999-08-26)<br>* the whole document *<br>---<br>-/-- | 1-32 | |

EPO FORM 1503 03.82 (P04C10)

European Patent Office

# PARTIAL EUROPEAN SEARCH REPORT

Application Number
EP 02 00 6257

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| D,A | POPOVICI T ET AL: "Down-regulation of striatal enkephalinergic (PPA) messenger RNA without prior apoptotic features following reversible focal ischemia in rat." BRAIN RESEARCH. NETHERLANDS 2 JAN 1997, vol. 744, no. 1, 2 January 1997 (1997-01-02), pages 185-187, XP002218347 ISSN: 0006-8993 * the whole document * --- | | |
| D,A | KITAGAWA K ET AL: "Delayed, but marked, expression of apolipoprotein E is involved in tissue clearance after cerebral infarction." JOURNAL OF CEREBRAL BLOOD FLOW AND METABOLISM: OFFICIAL JOURNAL OF THE INTERNATIONAL SOCIETY OF CEREBRAL BLOOD FLOW AND METABOLISM. UNITED STATES OCT 2001, vol. 21, no. 10, October 2001 (2001-10), pages 1199-1207, XP008009760 ISSN: 0271-678X * the whole document * --- | | TECHNICAL FIELDS SEARCHED (Int.Cl.7) |
| D,A | KOISTINAHO J ET AL: "Expression of beta-amyloid precursor protein mRNAs following transient focal ischaemia." NEUROREPORT. ENGLAND 4 NOV 1996, vol. 7, no. 15-17, 4 November 1996 (1996-11-04), pages 2727-2731, XP008009759 ISSN: 0959-4965 * the whole document * --- | | |
| | -/-- | | |

EPO FORM 1503 03.82 (P04C10)

European Patent Office

# PARTIAL EUROPEAN SEARCH REPORT

Application Number
EP 02 00 6257

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| D,A | SEYFRIED D ET AL: "CATHEPSIN B AND MIDDLE CEREBRAL ARTERY OCCLUSION IN THE RAT" JOURNAL OF NEUROSURGERY, vol. 87, no. 5, 1997, pages 716-723, XP000907116 ISSN: 0022-3085 * the whole document * ----- | | |
| | | | TECHNICAL FIELDS SEARCHED (Int.Cl.7) |

EPO FORM 1503 03.82 (P04C10)

European Patent Office

Application Number

EP 02 00 6257

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-32 (all partly)

European Patent Office

# LACK OF UNITY OF INVENTION SHEET B

Application Number
EP 02 00 6257

The Search Division considers that the present European patent application does not comply with the requirements of unity of invention and relates to several inventions or groups of inventions, namely:

1. Claims: 1-32 (all partly)

   Invention 1:

   Uses of (at least) SEQ ID NO:1 or of a combination of at least two polynucleotides of which one is SEQ ID NO:1 or of (at least) one polynucleotide sequence of a gene comprising SEQ ID NO:1 or of a protein encoded by a gene comprising SEQ ID NO:1 according to claims 1-3, 20-23, and 26, methods involving assessing the expression of at least one gene comprising SEQ ID NO:1 or of at least two genes of which one comprises SEQ ID NO:1 according to claims 4-19, a combination according to claim 24 comprising at least a gene comprising SEQ NO:1, a combination according to claim 25 comprising at least one amino acid sequence encoded by a gene comprising SEQ ID NO:1, a pharmaceutical composition comprising the nucleic acid sequence of a gene comprising SEQ ID NO:1 or the according polypeptide, a kit to detect a gene comprising SEQ ID NO:1, a vector comprising any nucleic acid sequence of a gene comprising SEQ ID NO:1, a host cell comprising said vector or any nucleic acid sequence of a gene comprising SEQ ID NO:1, an antibody that binds to a polypeptide encoded by a gene comprising SEQ ID NO:1, and a transgenic animal wherein at least a sequence corresponding to SEQ ID NO:1 is manipulated.

2. Claims: 1-32 (all partly)

   Inventions 2-34, 36-39, 41-76, 78-85, 87 and 88:

   Uses of (at least) SEQ ID NO:n or of a combination of at least two polynucleotides of which one is SEQ ID NO:n or of (at least) one polynucleotide sequence of a gene comprising SEQ ID NO:n or of a protein encoded by a gene comprising SEQ ID NO:n according to claims 1-3, 20-23, and 26, methods involving assessing the expression of at least one gene comprising SEQ ID NO:n or of at least two genes of which one comprises SEQ ID NO:n according to claims 4-19, a combination according to claim 24 comprising at least a gene comprising SEQ NO:n, a combination according to claim 25 comprising at least one amino acid sequence encoded by a gene comprising SEQ ID NO:n, a pharmaceutical composition comprising the nucleic acid sequence of a gene comprising SEQ ID NO:n or the according polypeptide, a kit to detect a gene comprising SEQ ID NO:n, a vector comprising any nucleic acid sequence of a gene comprising SEQ ID NO:n, a host cell comprising said vector or any nucleic acid sequence of a gene comprising SEQ ID NO:n, an antibody that binds to a polypeptide encoded by a gene comprising SEQ ID NO:n, and a transgenic animal wherein at least a sequence corresponding

European Patent Office

**LACK OF UNITY OF INVENTION SHEET B**

Application Number
EP 02 00 6257

The Search Division considers that the present European patent application does not comply with the requirements of unity of invention and relates to several inventions or groups of inventions, namely:

to SEQ ID NO:n is manipulated,

wherein n is chosen amongst 2-34, 36-39, 41-76, 78-85, 87 and 88.

3. Claims: 2-3 and 5-32 (all partly)

Inventions 35, 40, 77 and 86:

Uses of (at least) SEQ ID NO:n or of a combination of at least two polynucleotides of which one is SEQ ID NO:n or of (at least) one polynucleotide sequence of a gene comprising SEQ ID NO:n or of a protein encoded by a gene comprising SEQ ID NO:n according to claims 2-3, 20-23, and 26, methods involving assessing the expression of at least one gene comprising SEQ ID NO:n or of at least two genes of which one comprises SEQ ID NO:n according to claims 5-19, a combination according to claim 24 comprising at least a gene comprising SEQ NO:n, a combination according to claim 25 comprising at least one amino acid sequence encoded by a gene comprising SEQ ID NO:n, a pharmaceutical composition comprising the nucleic acid sequence of a gene comprising SEQ ID NO:n or the according polypeptide, a kit to detect a gene comprising SEQ ID NO:n, a vector comprising any nucleic acid sequence of a gene comprising SEQ ID NO:n, a host cell comprising said vector or any nucleic acid sequence of a gene comprising SEQ ID NO:n, an antibody that binds to a polypeptide encoded by a gene comprising SEQ ID NO:n, and a transgenic animal wherein at least a sequence corresponding to SEQ ID NO:n is manipulated,

wherein n is chosen amongst 35, 40, 77 and 86.

## ANNEX TO THE EUROPEAN SEARCH REPORT ON EUROPEAN PATENT APPLICATION NO. EP 02 00 6257

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2003

| Patent document cited in search report | Publication date | Patent family member(s) | Publication date |
|---|---|---|---|
| WO 9942610 A | 26-08-1999 | DE 19806431 C1 | 14-10-1999 |
| | | AU 2834299 A | 06-09-1999 |
| | | CA 2322068 A1 | 26-08-1999 |
| | | WO 9942610 A1 | 26-08-1999 |
| | | EP 1055003 A1 | 29-11-2000 |
| | | JP 2002504348 T | 12-02-2002 |

EPO FORM P0459

For more details about this annex : see Official Journal of the European Patent Office, No. 12/82

## SEQUENZPROTOKOLL

<110> Biofrontera Pharmaceuticals AG

<120> Multiple genes relevant for the characterisation, diagnosis and manipulation of stroke

<130> P10935EPalleSeq

<140>

<141>

<160> 88

<170> PatentIn Ver. 2.1

<210> 1
<211> 220
<212> DNA
<213> rattus norvegicus

<400> 1
```
gggagtcaat aaatcttatt agacataaca ggtacccaag gacgacagtc tactctaagc 60
tagctaccat atgcgtatca atgttgcaat ctcgttatgt ccagctgagg atcagaagcc 120
tgactattag atccgctata ccaggatata cctacgatcc cgtaggtgtc atgacgtatc 180
ataactagta ctgagacggg aaactgcaca taaaaaaaaa                       220
```

<210> 2
<211> 296
<212> DNA
<213> rattus norvegicus

<400> 2
```
tgaatcctag gatggatgag cgtgtctcat attataaccg tatacatcgt cattactcat 60
tgancgaaca gtataggtcg cgagaggagt ggtgctacca actatcaggc gcgttacttg 120
aggtgtcgat acgtctgcag ctcccgtagc ccgcagaatt atggtacatg aatcatatgt 180
attcgctaag gttctatccg tactggtaga ccagggctct ggaccatagt ctcatactca 240
aagtactaca tagactcaaa tgacagttca tgacgaacaa gtacacaaac aacaaa     296
```

<210> 3
<211> 287
<212> DNA
<213> rattus norvegicus

<400> 3
```
gtgggcgcaa ngaccantnc cccctaaaat ttntaaaacc ngnagggatt anatttnatt 60
cttctcagac aggtgaggnc ccactaagtc ctcggaagca aatagctgcg gtctcagaga 120
gcacangatc gttggccttg nattcatggc tgttgacagc tgtccatgcc atgatcatga 180
tttcgatatc aatgcttttc atnttagaat atgtanacat accgaagtga gtttgtgaca 240
ctattttaac ataaaatttc taacactcaa caaaaaaaaa aaaaaaa               287
```

<210> 4
<211> 91
<212> DNA
<213> rattus norvegicus

<400> 4

tactcttcca tacaagtcct atacacactt ttatgtacca ttgattgatc ctactgccct 60
tcttactttt cactcaaaaa aaaaaaaaaa a 91

<210> 5
<211> 258
<212> DNA
<213> rattus norvegicus

<400> 5
gtggaggctg agtgctacct atgatgtctt gaagtatatc acaattatct gtaatcctca 60
tcttcagact gcttccctcc accgtagact gtgcttcctc ctccagcgtg ccctgcatgg 120
cctagctcca gacgtcgaga gaggacagct atcgtctagg acagttctgg tgttaccctg 180
gagtccacgg gaggtgaact agtccagact gcctgagatg agtacagtat ctggcgtcac 240
caaaaaaaaa aaaaaaaa 258

<210> 6
<211> 200
<212> DNA
<213> rattus norvegicus

<400> 6
ttctcgcctc gtactttgct ggcttcctct cattcacaat ctgtcttatt gtgacccatc 60
tgagatttcc agtagtagtc tcattacaac agcgagatga cattcgtatc ctcatagata 120
taatgagtca caattctggt aatcatctaa attcacacag ttctcttact aaagtctctc 180
aatctcaaaa aaaaaaaaaa 200

<210> 7
<211> 347
<212> DNA
<213> rattus norvegicus

<400> 7
caagcggagg acatcgtcct catcttcata ggccgagcta caccaacatt attctatcta 60
atcgccctaa catctatcgt attcctaggc cccttatatc atatcaagtt accctgaatt 120
atactcaagc agcttcataa gcagaaacac atacttctat ccacacactt tcctactgaa 180
tcgcgagcat cctaccgccg cggtggcgag tatgaccaac tggatgcacc tccgtatgga 240
gaaaatttcc tcccacntaa cacgtagcat tctcgcagta tgatacattt gcgctgggca 300
atttgccagt agcagtgaac ttccatgcct acaggtataa aaaaaaa 347

<210> 8
<211> 183
<212> DNA
<213> rattus norvegicus

<400> 8
caaggcctca ctgtctgacg tcctccacag gtcctagctt cagctgaaat gttgcttctg 60
cagttttgtg tgcagttccc aactttctgc acagggacga tctttgtccc tgatcctgaa 120
gagtagaaat ggttcttaga aaagatttca aataaagtct gcacatcaaa aaaaaaaaaa 180
aaa 183

<210> 9
<211> 214
<212> DNA
<213> rattus norvegicus

<400> 9
gggggcggc agaggcaatt gtactactga tgttttatct agcttnagcc tgtgcccact 60
ctgatttgct cctgcaacca tacagctgtg gcatgtaatg aagtcctgtt gtgtgtccga 120

tgccccgagc ctctacattc aagcagcgaa tagagtgtga gagcaaggcc ttgtgaatca 180
gatgaaggat cgaagtgaaa aaaaaaaaaa caaa 214

<210> 10
<211> 159
<212> DNA
<213> rattus norvegicus

<400> 10
gtatctttct ctcancttac tgtcctatat ttcaatgaaa caacaaggca cttctctatt 60
tcatcattaa aagtggacaa cctatcattt tccttctcac caagaagagg atttgcctgt 120
gtacctaaag cttaggaatg ctaacaaaaa aaaaaaaaa 159

<210> 11
<211> 195
<212> DNA
<213> rattus norvegicus

<400> 11
aagtgcgagt gtaacattta gcatgacagt ctgtacctga tcactgtact gctcttacac 60
agtctggtca cagaatggga ggctagggtt gttgactgat ccagacccca actggcaact 120
tcatgtatgt tttcaaccat ccccttagtg gttctacttc aaaatagaag aaagacaaca 180
aaaaaaaaaa aaaaa 195

<210> 12
<211> 164
<212> DNA
<213> rattus norvegicus

<400> 12
ataccttgtg tcgcagctag tacgcttcta gacttcagag tctggatctc tatgcatccg 60
ctggtagagc tgtgcttgat agtacctcat aggctgcgta cgatcacgct ggccgcaagc 120
atatctgtca ttagatagga ggaatccaag aaaaaaaaaa aaaa 164

<210> 13
<211> 181
<212> DNA
<213> rattus norvegicus

<400> 13
aaacgctgcg aaccttctca ctccttacct acttttatct acgatcatac ttggaccacg 60
tgacataagt ctacagcact acagctctaa tcatgttgct tctgaacatc atgtacatta 120
atatttgtta ggcaattaat taaaataaac aatcttttta tgcgactaaa aaaaaaataa 180
a 181

<210> 14
<211> 110
<212> DNA
<213> rattus norvegicus

<400> 14
ccgtcttgga tgtgctgtct gactttctct ctatgtgaat agtcttactg ttcatctaca 60
tacattaaat taaaatgaag attcttatga ctcaacaaaa aaaaacaaaa 110

<210> 15
<211> 274
<212> DNA
<213> rattus norvegicus

<400> 15
atggagcgcg cgtcaacatg ctggtctatc gattacgctc gttcgtataa tccggtttca 60
ggttgctact gttacttaaa ctaggtcgtg gacgatagaa ggaccactgt cagcacagac 120
caactgggac attgctcaca tgatcagtgg ctttgaatga aatccagatc aagtgtccta 180
gagttgacgc ttggcccttg tgaacgtgac tgtagctggc tcaaaggcag acttttgtga 240
tcctaaatca gtccttcgaa ctgaaaaaaa aaaa 274

<210> 16
<211> 132
<212> DNA
<213> rattus norvegicus

<400> 16
cctcgacgcg gggcaaccga ccggcgccgt cagacctaga ataatgtcca ggttactctt 60
ctactagcag tactgtcata gctaggctct agctcaatta agaaatgtag gcgatgagaa 120
aaaaaaaaaa aa 132

<210> 17
<211> 139
<212> DNA
<213> rattus norvegicus

<400> 17
ctccggtcgc tctcccgaaa tactagcctc tatgttttga aactacgaga ncagangaca 60
ctaccaaagc acatgtagag gttctcgaaa cgataatgaa ataaacggta atgacttctt 120
cacacaaaaa aaaaaaaaa 139

<210> 18
<211> 276
<212> DNA
<213> rattus norvegicus

<400> 18
aaaagataca gtgagaagct ggagttggtc aattggaacc agatgttaaa aaattagaaa 60
acttgcttca gggtggtgaa gtagaagagg tgattcttca ggctgaaaaa gaactaagtc 120
tggcaagaaa aatgttgcag tggaagccct gggagccact ggtggaggag ccccctgcta 180
accagtggaa gtggccaata taatccccgt gtctgatgat ggatgtggat ctaatgtgca 240
attaaatgtt ctgtgatgct aaaaaaaaaa aaaaaa 276

<210> 19
<211> 129
<212> DNA
<213> rattus norvegicus

<400> 19
ccgtggagng gtacggaccg gagtccaaac tttntagttg gctgtntnca ttagatatgg 60
atcaccctga atgctcctaa taaacgtcgg aaagcctana ttatcacaac tccaaaaaaa 120
aaaaaaaaa 129

<210> 20
<211> 118
<212> DNA
<213> rattus norvegicus

<400> 20
cctacnaaac gagcgcggca cgtcgtgtcc tagtgtgtat tgtggctgtc ctctgagttg 60
gacatacttg tatcgtcttc aatcaagaca tgtctgtgta cactaaaaaa aaaaaaaa 118

<210> 21
<211> 154
<212> DNA
<213> rattus norvegicus

<400> 21
gctgctgcag cgactagtca ggactggaca tatagacagg gtgcgggtcc caatcgctcc 60
tgtgactgca ctggggcagc tgaagtgcag gaccttgcga ctagattgaa cgtcgtgatg 120
agactagtct cgtccttaaa taaaaaaaaa aaaa 154

<210> 22
<211> 103
<212> DNA
<213> rattus norvegicus

<400> 22
gggtggccgc cgcctcggat ctcatccttt tctctgacta catcattgca tttagcggta 60
gccgatgtag tatactaata ggcagacaac aaaaaaaaaa aaa 103

<210> 23
<211> 73
<212> DNA
<213> rattus norvegicus

<400> 23
tatctaagct caacttagct attatgctgt aggatgactg tgatactaca gttgttatca 60
aaaaaaaaaa aaa 73

<210> 24
<211> 107
<212> DNA
<213> rattus norvegicus

<400> 24
gcgcccgtcg ggtctgctct tcatatatca ttcttcttct ttattagttt taggttcctt 60
ctagcattca tgatatagct tacaaatatc ggaaacaaaa aaaaaaa 107

<210> 25
<211> 217
<212> DNA
<213> rattus norvegicus

<400> 25
actgcactaa cacgcactca ccttttgcct tctgccctca gccttcccgt ctgttcccca 60
tgttctttcc caaggaatct agccttgagt ccagccacca attgtctcac aatacacagt 120
gtggtattct tatcctctga aagagggctg aagggctgag aatggagtca ataaagcaag 180
gaagcaaaca tcctgtttct gccaaaaaaa aaaaaaa 217

<210> 26
<211> 151
<212> DNA
<213> rattus norvegicus

<400> 26
tccccccgag gccctaaggg ggctctgctc tcttctctct cttccttata tttatactcc 60
tagagcatac aaaaggacac tatctttaaa acaagaaata atatatatcc tatgaacata 120

tagtatagct aagtgcaaaa acaaaaaaaa a 151

<210> 27
<211> 200
<212> DNA
<213> rattus norvegicus

<400> 27
taacactgtg gacaggccaa ggcatatatt gcttcttctt agcattgcct acacacatct 60
gcagcgttcc tctaagagct ggccctgtga caggggtctg gggatttagc tcagtgttag 120
agcgcttgcc taggaagcgc aaggccctgg gattcggtcc ccagatctag aaaaaaaaga 180
acctaaaaaa aaaaaaaaaa 200

<210> 28
<211> 217
<212> DNA
<213> rattus norvegicus

<400> 28
ctggctgatg acccaacagg aagaaagacc ctggtgaacg ctactctctg ggacctccct 60
ttttgtgacc cgaaatgcct gtgcagtttt tcctgtccat cagccaaaaa tccacccccta 120
gcagagatcc aacaaacaga aagttcacct tgccacgcac tctgtcctct cctcttccat 180
gcattaaatt atgtttttag aaaaaaaaaa aaaaaaa 217

<210> 29
<211> 310
<212> DNA
<213> rattus norvegicus

<400> 29
tgtgactgca gtaacgcgcg gtcttgcggt ttactctgtg tcggggagat gtaccatggc 60
cacccagact ttaacagcac taaataactt agggagctgg gggagggaag ggcccaggac 120
tcgggccact cagcctaatg aaaccctgtt gctctgtcac cgtgtgccct ttggcctgac 180
caagtttgac actgggattc agtttaggcg ccagcctcaa gcacatccca gcagtggtac 240
ttcggagaaa tcagcatctg actgagcaga acaaatcgtc aggtgcctgg agcaaaaaaa 300
aaaaaaaaaa 310

<210> 30
<211> 211
<212> DNA
<213> rattus norvegicus

<400> 30
tctcctgcct ttgtcctgtc ttctctctat ccctctctat acgtctctgt atattttacc 60
ggaccttccc gatcnatgct gtgtacgaaa tacatggtga cgcctaggca attcctatga 120
tacttctacg tatgctagat gaagcttata cgtacttaga tagataactt acaaaataaa 180
gtcttgatat cagtagacaa aaaaacaaaa a 211

<210> 31
<211> 410
<212> DNA
<213> rattus norvegicus

<400> 31
gtgactgtga acnggcgtcg tntcgatgca cggtccgtgg natctcttna aggaggaccc 60
ccctacntat cggactgtcc ncattncagc tggctcctgc ccangtgatt taatataaca 120
ttcattggnn tgatcgataa tgttanggta caccncgtgg cacattgcat nggagtgaag 180
tgaacaaggc tgtcaccaaa tcacacacgt tttaataaga aatgtttact aagggagcat 240

ctttggactc tctgttttaa aaccttgtga accatgactc ggagccagca gagtaggctg 300
tgtctgtgga cttgagcaca ccatcaacat tgctgttcag gaaattataa tttacgtcca 360
ttccaagttg taaatgctag tcttttattt ttttttccc aanaaaaaan 410

<210> 32
<211> 341
<212> DNA
<213> rattus norvegicus

<400> 32
ccaactcact gggacactga aatccntatt gagtgtaagg cgtatggtga gaacattggg 60
tacagtgaga aagaccgttt tcagggacgc tttgatgtaa aaattgaagt taagagctga 120
tcacaagcac aaatctttcc cactagccat ttaataagtt gaaagaaaaa gatacacaaa 180
cctactagtc ttgaacaaac tgtcatacgt atgggaccta cacttaatct ctatgcttta 240
cactagcttc tgcatttaat aggttagaat gtaaatttaa agtgtagcaa tagcaacaaa 300
atatttattc tactgtaaat gacaaaagaa aaaaataaaa a 341

<210> 33
<211> 411
<212> DNA
<213> rattus norvegicus

<400> 33
ggtgactcgt gcgctgccgg tggtgggagt gagcatgctc aacgtttgcc tgaagtcgcg 60
acacgaagag cacgagagac ccgagttcgt cgcctacccc catctccgca tcaggactaa 120
gcccttcccc tggggagatg gtaaccatac cctcttccac aatcctcaca tgaacccgct 180
tccgactggc tatgaagatg agtaaagaga acctggctct tcgcccaggc gacaagggac 240
cacagcactg atttggaccc tgactcttgt gtgtggacca cgaaagccca ttggatgctc 300
agctcatctt tcctttatca gatggtgacc attactttgc tcctccatcc ctttgctcgt 360
aagaggagat ggcttaaata aataacttga actgagaaaa aaaaaaaaaa a 411

<210> 34
<211> 118
<212> DNA
<213> rattus norvegicus

<400> 34
cggacgaata gatcgtaacg acttactctc tgctcttaag gacgaactgc tgtccccacg 60
cgaactaagt tcaagtaaga gccagccggt agcgatacga acaagaaaca caacaaaa 118

<210> 35
<211> 186
<212> DNA
<213> rattus norvegicus

<400> 35
cngcnaagct gtgattcagg ggttgctgta ttcttttgag tctggaagct cagtattggt 60
ctctgtggct atgttgttat catgctgaaa cagtctgtta cctcatccct tctgacaaaa 120
cgtcaataaa tgcttattng tatataaata ataaacccgt gaacccaact gcaaaaaaaa 180
aaaaaa 186

<210> 36
<211> 205
<212> DNA
<213> rattus norvegicus

<400> 36
ccccgggaga ctagcacact gctgtcgtca gtacctccag aactgaggga cccagcccag 60

gaggacagga tcttgccaaa gcagtagctt cccatttgta ctgaaacagt gttcttggct 120
ctataaaccg tgttagcaac tcgggaagat gccgtgaaac gttcttatta aaccaccttt 180
atttcattca aaaaaaaaaa aaaaa 205

<210> 37
<211> 202
<212> DNA
<213> rattus norvegicus

<400> 37
gcaggaccgc agctttatga ctgcatgttt ggatgttagc tctcgtctta tgagtcatcc 60
actgcggatg cacttggata tgcttattgc gtattcctca tctgtacttg tttgtgcgat 120
atcgttattt cgtgactatg tattccagct cttgtgtctn catcgatnat cgactgttga 180
acactcaaga aaaaaaaaaa aa 202

<210> 38
<211> 200
<212> DNA
<213> rattus norvegicus

<400> 38
aaagggacta actantgatt attagactgg catttactta gataaagttt gtggggccag 60
attctttcca gacatgtgat tcacatacag gaatttctta cacatccacc tctcccctcg 120
tgcagtatgg ccctgatgct taaaatctga gtaactaaga catctttgaa gatttaaacc 180
aagtaatgca aaaaaaaaaa 200

<210> 39
<211> 146
<212> DNA
<213> rattus norvegicus

<400> 39
agccagtaac gacagctatc taggaccaag ctctgaaatt ggcggctcag atcacagctt 60
catacccatg gtactagaaa tagtgcctct aaaatatttc gaaaactgat cagcttctat 120
aattcaaacc aaaaaaaaaa aaaaaa 146

<210> 40
<211> 139
<212> DNA
<213> rattus norvegicus

<400> 40
caaaatacgt taatatgaac antcactacg cctttacgta anatcatgac agccaggtgg 60
ccttgtccca agcaccactc tggccctctg gatggccctt gcttaataaa tgattctcca 120
agcaaaaaaa aaaaaaaaa 139

<210> 41
<211> 234
<212> DNA
<213> rattus norvegicus

<400> 41
atacagncca cacttaactc acccttacac tcaaatcatt atgtcgacag atattgactt 60
accgaagtgc tattagacaa agagtcccgt caaggagagt gcctgaaagg agaaatgatg 120
aaagtactaa ctagagcaga taattacacc gtcgtacctt ttagctatat atgctattag 180
acttggatga attcattcat agacaaaatc cattaaacaa agaggctcga aaac 234

<210> 42
<211> 291
<212> DNA
<213> rattus norvegicus

<400> 42
ggacatgaca tgaccagtgt atctactgtg gtttctgcca ggaagcctgc cctgttgacg 60
ctatcgtgga gggccccaac tttgagttct ccaccgagac gcatgaggag ttgctgtaca 120
acaaggagaa gctactcaac aatggtgaca agtgggaggc cgagatcgcg gccaacatcc 180
aggctgacta cctgtatcgg tgaccgggcc accggtgacc ttgccacctg gccagccttg 240
tggcccctat agcccataaa gaaactctga tcccaaaaaa aaaaaaaaaa a 291

<210> 43
<211> 113
<212> DNA
<213> rattus norvegicus

<400> 43
cggttnccgc gggctgtagc gaactcgagc ggaccgtctc tcccggtaca gttattattt 60
atgtcactgg ctccttatta aagatcttta accctcaaaa aaaaaaaaaa aaa 113

<210> 44
<211> 197
<212> DNA
<213> rattus norvegicus

<400> 44
tggggntcat tggcaataat gacctaggtn ttaacagtct tcctaagcat anaatttang 60
ctaaagcaag cccgacacct aanatcgacg tctatcgtaa nccantcacn cgtttcagng 120
acnctcaacn cgtactactt agacncaata gncgggnctc gatcgtntca ggatagcgtg 180
gtcanncgcg tatcacn 197

<210> 45
<211> 206
<212> DNA
<213> rattus norvegicus

<400> 45
cacgttannt atggagcgcg ncgtactgac ttactcagtt tgcgttccct ttcctcgtta 60
tgccttactg aactatgtac ttgacatgta gtgctacact tgggagagtt gttagcgctc 120
tgctcccact ctctgtctac tcttcttgca tgtgtgggta ataaaggcgc ccggagaggg 180
caagtgacta aaaaaaaaaa aaaaaa 206

<210> 46
<211> 146
<212> DNA
<213> rattus norvegicus

<400> 46
tgggacatgc tgctacatca tgtctatttt ttactactac atccttataa caattacgtt 60
tccaacttac gttactactc catttatgca caggttcgag ataaattaat ttttgtaata 120
tggacactga aaaaaaaaaa aaaaaa 146

<210> 47
<211> 181
<212> DNA
<213> rattus norvegicus

<400> 47
cagagacgcc gttcttgatt tattctcgcc cttcattccc atggcctgct gtctatgagt 60
acaaatagta atggtggacg tgactgcttg ttgccaaact ggaacatgtt ctgtaggggt 120
ttactggcat ggtatcattc ctaggaagaa gaagagggaa aaaaaagagg aaaaaaaaaa 180
a 181

<210> 48
<211> 272
<212> DNA
<213> rattus norvegicus

<400> 48
cagccagcag gaccaaactc aatcacagta gataaatact atgtccctcc acgatgcgtt 60
gtcacgcgat gaggacaaga gaaatcgacg cgaccgaagt catgacagcg cgcgaacagc 120
ccaacgcgtg cacgcgaccc gacgccagga cgagagccga caagctgatc accaggagag 180
acacggagat aggatgcgcc aaggcagagc gaggacggcc agcaccagaa ggaagcgtgc 240
gcggtaggag tgactatgag cactgtagna gt 272

<210> 49
<211> 73
<212> DNA
<213> rattus norvegicus

<400> 49
atctgcttaa agtctttaat ttgtactatt tcttcaaata aagaattttg gtacccaaaa 60
aaaaaaaaaa aaa 73

<210> 50
<211> 125
<212> DNA

<213> rattus norvegicus

<400> 50
tccaaaatat ttctgtgaag ctcagtcctc tgttcctctt ttttattttt ttttgttcct 60
ctgtagacat gatggaggag tttactagaa aataacgtga gatgggagca ggaaaaaaaa 120
aaaaa 125

<210> 51
<211> 365
<212> DNA
<213> rattus norvegicus

<400> 51
aaacatggcg acgatgcaca gaccgaataa ctgtgtctta atcagagacc ctccctctag 60
cacccagcgt gccatctcct acctaggaac gagcaacttg ctcaaaggcg taggtgactt 120
gtgggccatt catctacaag tcctgatgga acaaggccca agactaaggg atgtaagaac 180
gacccattga tataacgtta ttagtcccag ccaatgtctt cggtgatatt cccagttgca 240
cttccctacg ttcgagaaca atccaacgag acactccaga ctcggtctgt gttaatgtcg 300
catagctccc cttttgtaca acataaacat tatactgtga tgtgaacaac taaaacaaaa 360
aaaaa 365

<210> 52
<211> 175
<212> DNA
<213> rattus norvegicus

<400> 52
tggaggagta cctagtatta ttgacatcag tccgccaggc gcaacaatta catttgttat 60
tctactgcgt acttacaggt acttgaattg gtcgaagtcc ttaattcaat gcctatgtat 120
tcacccttct agtaagcctg tagctacatg actcacacat acaaaaaaaa aaaaa 175

<210> 53
<211> 182
<212> DNA
<213> rattus norvegicus

<400> 53
cacagcgcct ctctgttcac cacggcgtag ttacgatata tctctagcta gttcttttac 60
attagttgac gtgtacttcc tcttgtgcag actgccgctg tccttgctcc actgatgggc 120
ctgagcagtg ggtaagaact ccgtatgtaa ttgccgatac taaccagcaa aaaaaaaaaa 180
aa 182

<210> 54
<211> 101
<212> DNA
<213> rattus norvegicus

<400> 54
gggacgagcn canacgcgat ttccacgtta tcctcattct cataccagtc tctggcaata 60
gtagaagacc agatgttaca atgaaattaa taaaaaaaaa a 101

<210> 55
<211> 123
<212> DNA
<213> rattus norvegicus

<400> 55
ccgtccgtgg ggacgactat tatattttat tctttatctt ttctttctct ccagagacga 60
tgcggccgag agaactggag cctcctatat cagtaagtgc tcgcagccca aaaacaaaaa 120
aaa 123

<210> 56
<211> 76
<212> DNA
<213> rattus norvegicus

<400> 56
ctacgaaagg acaagagaat tggagcctcc ttaccataag tgctcccaac caatttatga 60
aaaaaaaaaa aaaaaa 76

<210> 57
<211> 261
<212> DNA
<213> rattus norvegicus

<400> 57
ccgngaaacg gctgatgtgt catctttgct tcctcaggta ataagtctta acaggcccgc 60
catcgttggt ctcatcaggt cctgctctct agaagtcgga tcagaaacag acttgaaaat 120
gtgccgaaga attgcatctt agggtccgac gaaaaatgta tatagttgct gagtcctgag 180
actcatgtgt gtgcacaaga aaacctggtt ttcccttaga atttacaact aaaggaagta 240
acaagaaaac aaaaaaaaaa a 261

<210> 58
<211> 269

<212> DNA
<213> rattus norvegicus

<400> 58
tctgcggtgc ccaccagtat actggagacg tacttgtaac agggcccagg catactctag 60
gtcctccatc acggacctgc ttctctcaga acgtcgagat cagaaacacg acttgaaaag 120
tgtgccggaa gaatttgcat cttagaggtc cgacaaaaaa tgtatatacg tttctgagct 180
cctgacgact catagtgtgt gcacaacgaa aacctggatt ttcccattag tatcctacac 240
ataaaggcaa gtaagcaaga acaaaaaaa 269

<210> 59
<211> 277
<212> DNA
<213> rattus norvegicus

<400> 59
cggggtgttt gtttcattct gtctaatgtg aattttgtgc ctgctcctat ctgctcccct 60
gtacccccag cttcctgctt ttctcccaca ttctgaactg tccagtcctg tgatgtgtct 120
gaccttggac tcttcctgaa ggagctccct aggcaggaat atggtcccct attcagacac 180
taggccaggt gtgactgggg ctctcttagt ggccctctta gtggatgtgt tggcaacctt 240
aataaatcta gtggcagtgg caaaaaaaaa aaaaaaa 277

<210> 60
<211> 228

<212> DNA
<213> rattus norvegicus

<400> 60
cgggctgcat gacatatnga ctatccttga ctgcatctgt tccatcagca taatggaggt 60
catccccagg catggatccc acctttacac taggtcttaa cctagttacg tatacctact 120
cacgttatca cacctagtac ctcacgtagc taatacaagt ttgatactac atgacgcgtc 180
ccatatcttn tnatacgctt aggcncntct cggtcngagt agatcaaa 228

<210> 61
<211> 119
<212> DNA
<213> rattus norvegicus

<400> 61
gtttgtcttt ctttcctttc cttctgtttc ttttatccta ctgtgaccca cttatgcaaa 60
accacaggta tactcactac tatctcatat ttctagcgct acgaaaaaaa aaaaaaaaa 119

<210> 62
<211> 117
<212> DNA
<213> rattus norvegicus

<400> 62
aagaaacgga gggagaccgg agagctgccc gctactgtcg gtaatctatc tcagtgtgga 60
caagatatgt agggtagtgt gtagtatgct agtgtactag gccaaaaaaa aaaaaaa 117

<210> 63
<211> 217
<212> DNA
<213> rattus norvegicus

<400> 63
agcactcctc cacacaccac gactccgtct ttaagccgac gttctagcac cagtatccca 60
aactcaccct actcatagag tcatagaagc caatgcctaa tcccattctg agcaacatat 120
acactagaac agtctatatc tccgccacag tcattagatg aacaaaggct aatactgaga 180
ttagatagac cttgtcaagg ggttggcaaa acaaaaa 217

<210> 64
<211> 269
<212> DNA
<213> rattus norvegicus

<400> 64
cccagtttcg ttcgattaag tcagcgcacg ttcggagggt agtactatct acccagtaga 60
gatcctatgt gcaatgcagg aagaactata gctgctgtag tgtagcgtgt acttcagtat 120
acatcctgca taccacaaca accccaccac gcacccccgc ggttcataac acccgagggt 180
caccgtggcc acaacttcgt gtcacgctat tcttgttaca tctggccaac attactcacc 240
ccaccatata caactcgtta tccccacaa 269

<210> 65
<211> 162
<212> DNA
<213> rattus norvegicus

<400> 65
tctgtatacc gcattagagg cttgtagtat ccatccctct gtcacactta gtattcacat 60
ggtgcgtact ttcgtgtata ttcaaactct ctgttaccct gacaaaatcc aaacttttgc 120
tgttcacatc acatatgatg cacaattcaa aacaaaaaaa aa 162

<210> 66
<211> 200
<212> DNA
<213> rattus norvegicus

<400> 66
cggggaagaa agcaggctgc caggtgtgtc ctgtctcatg atacagaaat ggtttccttt 60
cggttattat tctggagcct caaatagcat tataacgttc tgtgattatg attgccttta 120
tctttaatta tttctgtaac actccacact agtcttggga aaccggcccc ttattttaga 180
gagaaaaaaa aaaaaaaaaa 200

<210> 67
<211> 182
<212> DNA
<213> rattus norvegicus

<400> 67
tggtgnttct gtcctggact cctccagaca gcatggagga attctgtttt aagattatct 60
tggagtttaa gtgattgccc tggaaaaatg aaatgtacca accatgtgga aatgacagct 120
acgtgtacat tatgtatgaa atgccaaata gagggacaag ttgggagata aaaaaaaaaa 180
aa 182

<210> 68
<211> 207
<212> DNA
<213> rattus norvegicus

<400> 68
cgtgagttaa tgagcttcga gccttaccac tctcccttta aatcaggaag tgcataggct 60
aattactatc agtcccgata tatttgttaa aggaacacct acaagatcct tactggtgac 120

cttctgtgag acactagttt gaggcactac atgtgtactt gaaaataata aagttgcatt 180
tctttatgaa taaaaaaaaa aaaaaaa 207

<210> 69
<211> 268
<212> DNA
<213> rattus norvegicus

<400> 69
gtgtgtgatg agattatgtg atggggaggc tgaacacagt tctatatttt agtatttttt 60
agtaatttgt actgtatttt tccttgcaga tattgaagtt atgaaccatt tactttgtgt 120
tctactgagt aagatgactt gttgactgtg aaagtgaatt ttcttgctgt gttgaacaat 180
caggactgcg ttcacttgag atccttgtag aataagcaca ggccgttttt cactttggta 240
ttgatacaat gtaaaaaaaa aaaaaaaa 268

<210> 70
<211> 285
<212> DNA
<213> rattus norvegicus

<400> 70
gacatacttc acgcgaatat atgactggat gtacagttac ccagcacgtg ttcctcctcc 60
tcctcccatt gctcgagctg tggtgccttc caaacgccag cgtgtgtcgg ggaacacctc 120
acgaaggggc aaaagtggat tcaattcaaa gagtggacaa cggggatctt cttccaaatc 180
tggaaagttg aaaggtgatg accttcaggc cattaaaaag gagctgactc agataaaaca 240
aaaagtggat tctctgctgg aaagcctgga aaaaaaaaaa aaaaa 285

<210> 71
<211> 158
<212> DNA
<213> rattus norvegicus

<400> 71
atggacgacg gtgaatgcag gactgggctg tcgtgtagca tcaccaccac cttcacatag 60
taacccccatc gttggagcac agaggaagga agatcatcag ngcagcttgg gctacttagc 120
agaccctgtt ccaaaaaaaa aaaaaaaaca caccacag 158

<210> 72
<211> 144
<212> DNA
<213> rattus norvegicus

<400> 72
gagccgaggt gatgcctcgt agtctctctc tctgttgntc tcttctggtg gtgacggggt 60
tatcccttcc cagttgtatt atattcctgt ggggcacatc ccaaagtatt aaaagtagct 120
tagtaattca aaaaaaaaaa aaaa 144

<210> 73
<211> 250
<212> DNA
<213> rattus norvegicus

<400> 73
aggcgcacta gagatcagaa ttacgctctc ctttccacat cagatgtgaa aactgtgatc 60
acaacagtag tacagtttgg tttcattgaa aataaactga attctaaagc atgctttttc 120
actggtccct ttgcttttgc tacttcgaga cctcttggtt tatataacac tgaggttaag 180
attaaagctt ttcagaatgc caggcaaaga ctagagtttt gacccgaaca cacacacaaa 240
aaaaaaaaaa 250

<210> 74
<211> 122
<212> DNA
<213> rattus norvegicus

<400> 74
ggaaagtagt ggtggtagcg tgtanttctt atattatgtg ttacatatgt gtcgtctata 60
tatataagta catcccttag aactgctagg accatctcag aactgcacaa aaaaaaaaaa 120
aa 122

<210> 75
<211> 157
<212> DNA
<213> rattus norvegicus

<400> 75
ggtccanngg taatannttt acctngtgtt tattgnngta tatagctcac tccgttacgt 60
tcttgtctag gcgtggccct anacggggac ttgagtaaat caccgtgatg ctcatgcccg 120
atgtaaggga taagagatgt acaaaaaaaa acaacaa 157

<210> 76
<211> 107
<212> DNA
<213> rattus norvegicus

<400> 76
gggattggtg tggcttcttg tntaatctgt ctattcttct ggtggtgatg tgttgtgtag 60
tatatgttac gcagtcatta gcacgggagg taagcaaaaa aaaaaaa 107

<210> 77
<211> 254
<212> DNA
<213> rattus norvegicus

<400> 77
tgcgacgtga gacacctgct ccctctctgg tgtatttata catacgtgga cagaaacaac 60
tcgtctgttt attgactcac cctcggtctg agacagcggt gctgtaacag aagtagatgc 120
ctgaactcga gtcaatatac aaatcaataa tgtatgctct ctttctctct ttacattctg 180
gtcactacac tacagttatg aaatgggatg ctagtgtctt ggacagccca aaaaattggc 240
cgctcgtaac aaaa 254

<210> 78
<211> 208
<212> DNA
<213> rattus norvegicus

<400> 78
aggcatccta tctgctaggc tactgtgaat ttgctctcca gtctacctgc ctgagcagtg 60
tgcatctact attgacagga gagtctgctc ccagacactc tgcctttccc tccacaatcc 120
tcgtcattcc gcatcctcat ggtaaactag gtcacgacgg cagtgcaatg cgtaaataac 180
cgaggtggac tttcgaatgt acaaacaa 208

<210> 79
<211> 300
<212> DNA
<213> rattus norvegicus

<400> 79
aacggtagtt gaccagtacg gattgactga tccttgtgtt ttcctcttgc aattggcccc 60
aaacatgtcc ctggcaagtg gagtgaaggc tttttgtcta aagatgacta gggtcagctc 120
aggagttgtg ggggagggcg ttttcatctt ccccgttgtc acttagaggt ttcgaactct 180
ggtgtaaaga ggccgtttat ctttgtaaac acaaaacatt tttgctttct ccggtttcat 240
gttaatggcg aaagaatgga agcgaataaa cgtttcactg actttttgaa acaaaaaaaa 300

<210> 80
<211> 192
<212> DNA
<213> rattus norvegicus

<400> 80
cacgagagga gaacatttaa cttttggtca gcagaatgaa ggaagcaaag agaagcgcca 60
ggaacagatt gtcaagagca cgtaggctgt cttcgctgag agcttctact tctaaatctg 120

agtccagtca aaaataagtc tttaaagagt caacaaataa ataatgagca ccttgaaaaa 180
aaaaaaaaaa aa 192

<210> 81
<211> 300
<212> DNA
<213> rattus norvegicus

<400> 81
gcaaaataac tgactgctca cccccgcttc tcatcaccag gacagcaggt cgagaaatgt 60
ctttccttgc acttgcttct ggggtttgtg attcttctaa ttttccccct tgctgtatct 120
ccctccttac cccctccact cgttccctgt ttctgtttat gcggaaatgg cagaaacgct 180
tgagaaatgc gaatgtgtaa gtggactgga agtttatagt ctggattttc aattttactt 240
tgtactgtac atctttttac tttagaattt gcaataaagg gttacgtcaa aaaaaaaaaa 300

<210> 82
<211> 328
<212> DNA
<213> rattus norvegicus

<400> 82
ggtatcgtga gagcaaaata aactgtactg ctccacccac cgcttctcat caccaggaca 60
gcaggtcgag aaatgtcttt ccttgcactt gcttctgggg tttgtgattc ttctaatttt 120
ccccccttgct gtatctccct ccttaccccc tccactcgtt ccctgttctg tttatgcgga 180
aatggcagaa acgcttgaga aatgcgaatg tgtaagtgga ctggaagttt atagtctgga 240
ttttcaattt tactttgtac tgtacatctt tttactttag aatttgcaat aaagggttac 300
gtcaatcttg tttccaaaaa aaaaaaaa 328

<210> 83
<211> 186
<212> DNA
<213> rattus norvegicus

<400> 83
aacgcanaag agctancgag caacccgant gtacttcacc ggagactggg tgaccggacc 60
tcactgcggc atttctgcag ggccatctgg acatggcgcc taggtcagcg aggtgcagca 120
caggcttgac gtattcgagc gattgcccta taagtccgac ggctcaatta aacacacaaa 180
caaaaa 186

<210> 84
<211> 370

<212> DNA
<213> rattus norvegicus

<400> 84
ctggcggtat acgtctcaat cccttagtta tnttatctaa gtctgggtgt gtacctagtg 60
tcacacatga ctagcatatg acctaactgt atgggcagtc tatggatttc ggacttccgc 120
tcctccctcc ctcctgatcc ctcgctggtt cgtggcatga tccgtaatag actctggtta 180
aaatccatcg tcggtggttg cagcttcctc atatacgcga gctgtgagtg gtattaggtc 240
atgcacccct cacagtgcat ggtcgcgata ctctacgcta atccatcata ggcttcggga 300
ttaacagctt cttcacgttg ctgagacagc aagaaacaaa caatatggct cctctcccaa 360
aaaacaacac 370

<210> 85
<211> 441
<212> DNA
<213> rattus norvegicus

<400> 85
cgaatacatc atggcgtctc tcatagagtt tgatggcgaa gacatcacga ctagaatcat 60
gaactacaaa accgcccgct ttgacagccg cttccaaacc agaaccagac taagaactgc 120
tggcagaact acctggactt ccaccgctgt gagaaggcaa tgacggcaaa gggcggtgac 180
gtctccgatg tgcgagtggt accggcgagt gtacaagtcc ctctgccccg tctcatgggt 240
ctcagcctgg gatgaccgca tagcagaagg cacatttcct gggaagatct gacctggctc 300
cgcccacctc tcctctgctc tttgaccttc tccccgatag aaaaggggga cctcagtata 360
tgatggtccc tatcctggga ccctgaatca tgatgcaact actaataaaa actcactgga 420
aaggttacaa aaaaaaaaaa a 441

<210> 86
<211> 412
<212> DNA
<213> rattus norvegicus

<400> 86
gtgcagctag tcacttctag cgactgtggc aggctttgat ggcccaatgc agttctctgg 60
agaaaactac ctttccccaa ggcatctgca cccattgaca atggtaatgt gcccatctct 120
ccttggtcct gccctcaacc gatgcttttc cagtcagggt tttgtttttt gttttgtttt 180
gtgtacctca actgagttat gaagatttgt actggtttta cagatcatct catcgtatgg 240
attagaacaa gcttcgtggt cagtttgctg ggtgaccggc agacaccaca atcaaactag 300
tctgggaaaa acctgctttt ttgttgtagg tgccacgtaa ccctgtcagt ttaacaagga 360
atgaccgtgc caataaacca attctccctc tgcttgaaaa aaaaaaaaaa aa 412

<210> 87
<211> 149
<212> DNA
<213> rattus norvegicus

<400> 87
cgttaggcgc agtcgtacag gcgtactgtt tctctatcta cttgctgctc gggtcagatg 60
cgacttcaaa tctagatggg acgccgtagg tccatgtatg ctaatgaggg gtgagctagt 120
attacttatt atcaggaagc aaaaaaaaa 149

<210> 88
<211> 207
<212> DNA
<213> rattus norvegicus

<400> 88
actagtagag ttcgaantag tctcgttaga tccggaatgt acctcgccga gatcagactg 60

```
ggaaaatgac tacctttctc acaacgaaaa cagtcccggt ggccctctgc cctggacctt 120
tgggattctg ggactagttc tgttctctag tggccaattg taactcgtgt acaataaacc 180
ctcttgctgt caaaaaaaaa aaaaaaa                                     207
```